(19) Europäisches Patentamt
European Patent Office
Office européen des brevets

(11) Veröffentlichungsnummer: **0 268 915 A2**

# (12) EUROPÄISCHE PATENTANMELDUNG

(21) Anmeldenummer: **87116505.6**

(22) Anmeldetag: **09.11.87**

(51) Int. Cl.[4]: **C07D 401/06**, C07D 417/06, C07D 403/06, C07D 405/06, C07D 413/06, C07D 409/06, C07D 213/75, C07D 213/53, C07D 233/88, C07D 233/61, C07D 239/42

(30) Priorität: **21.11.86 DE 3639877**

(43) Veröffentlichungstag der Anmeldung: **01.06.88 Patentblatt 88/22**

(84) Benannte Vertragsstaaten: **BE CH DE FR GB IT LI NL**

(71) Anmelder: **BAYER AG**

**D-5090 Leverkusen(DE)**

(72) Erfinder: **Wolf, Hilmar, Dr.**
**Haus Gravenerstrasse 105**
**D-4018 Langenfeld(DE)**
Erfinder: **Abbink, Jan, Dr.**
**Krutscheider Weg 49**
**D-5600 Wuppertal 11(DE)**
Erfinder: **Becker, Benedikt, Dr.**
**Metzkausener Strasse 14**
**D-4020 Mettmann(DE)**
Erfinder: **Homeyer, Bernhard, Dr.**
**Obere Strasse 28**
**D-5090 Leverkusen 3(DE)**
Erfinder: **Stendel, Wilhelm, Dr.**
**In den Birken 55**
**D-5600 Wuppertal(DE)**
Erfinder: **Moriya, Koichi**
**5-7-11, Ueno**
**Taito-Ku Tokyo(JP)**

(54) **Trifluormethylcarbonyl-Derivate.**

(57) Es werden neue Trifluormethylcarbonyl-Derivate der Formel (I)

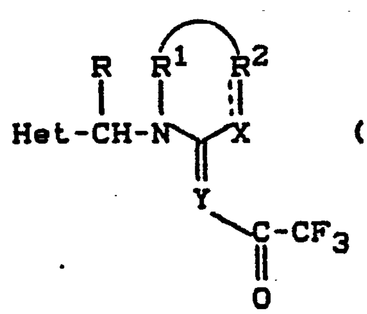

(I)

bereitgestellt,

EP 0 268 915 A2

in welcher
Het für gegebenenfalls einfach oder mehrfach substituiertes Hetaryl steht,
R für Wasserstoff oder $C_1$-$C_4$-Alkyl steht,
X für Sauerstoff, Schwefel, Stickstoff oder für die Gruppierungen $>$NH oder $>$C-$R^4$ steht,

wobei
$R^4$ für Wasserstoff oder Alkyl steht,
Y für Stickstoff oder den Rest $>$C-$R^3$ steht, worin
$R^3$ für Wasserstoff oder die Gruppierung -CO-$CF_3$ steht und
$R^1$ und $R^2$ gemeinsam mit dem angrenzenden Stickstoffatom und dem Rest X für einen heterocyclischen Rest der Formel

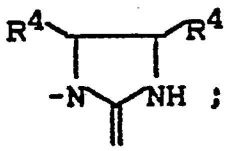 ; 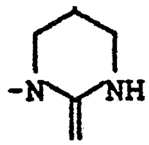 ; 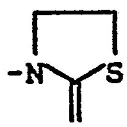 ; 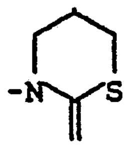 ;

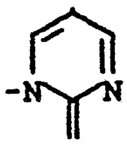 ; 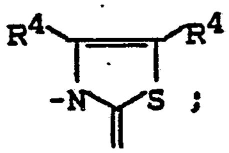 ; 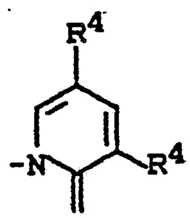 ; 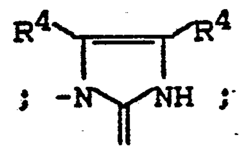 ;

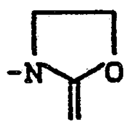 oder 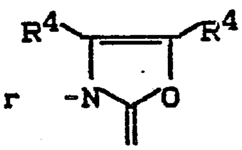

stehen, worin
$R^4$ die oben angegebene Bedeutung besitzt.

Die Verbindungen (I) können nach verschiedenen im Anmeldungstext angegebenen Verfahren hergestellt werden und besitzen eine stark ausgeprägte insektizide und ektoparasitizide Wirksamkeit.

## Trifluormethylcarbonyl-Derivate

Die vorliegende Erfindung betrifft neue Trifluormethylcarbonyl-Derivate, Verfahren zu ihrer Herstellung und ihre Verwendung in Schädlingsbekämpfungsmitteln, insbesondere als Insektizide. Darüber hinaus besitzen die neuen Verbindungen eine stark ausgeprägte ektoparasitizide Wirksamkeit.

Es ist bereits bekannt, daß bestimmte Thiazine wie z. B. Tetrahydro-2-nitromethylen-2H-1,3-thiazin insektizide Eigenschaften aufweisen (vergl. US-PS 3 993 648).

Es wurden nun die neuen Trifluormethylcarbonyl-Derivate der Formel (1)

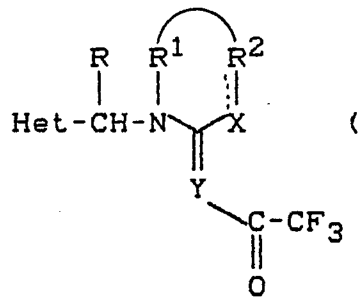 (I)

in welcher
Het für gegebenenfalls einfach oder mehrfach substituiertes Hetaryl steht,
R für Wasserstoff oder $C_1$-$C_4$-Alkyl steht,
X für Sauerstoff, Schwefel , Stickstoff oder für die Gruppierungen

>NH oder >CR⁴ steht, wobei
$R^4$ für Wasserstoff oder Alkyl steht,

Y für Stickstoff oder den Rest >CR³ steht, worin $R^3$ für Wasserstoff oder eine Gruppierung -CO-$CF_3$ steht und
$R^1$ und $R^2$ gemeinsam mit dem angrenzenden Stickstoffatom und dem Rest X für einen heterocyclischen Rest der Formel

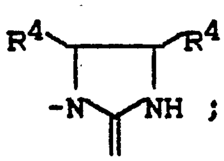 ; 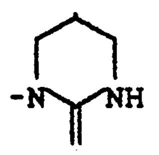 ; 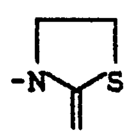 ; 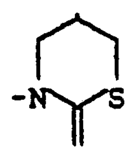 ;

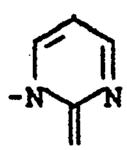 ; 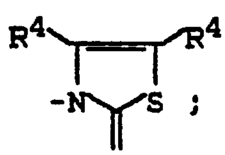 ; 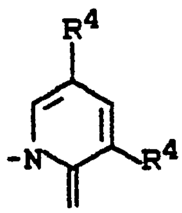 ; 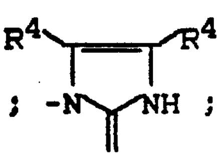 ;

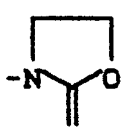 oder 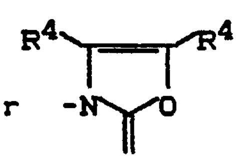

stehen, worin
$R^4$ die oben angegebene Bedeutung hat,
gefunden.

Weiterhin wurde gefunden, daß man die neuen Trifluormethylcarbonyl-Derivate der Formel (I) erhält,

wenn man

(a) zur Darstellung von Verbindungen der Formel

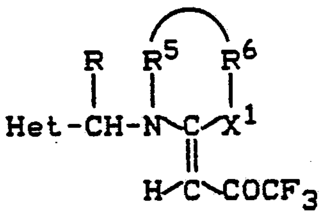

(I')

in welcher
Het und R die oben angegebenen Bedeutungen besitzen,
$R^5$ und $R^6$ gemeinsam für einen gegebenenfalls durch $C_1$-$C_4$-Alkyl substituierten $C_2$-$C_3$-Alkylen-Rest stehen und
$X^1$ für Sauerstoff, Schwefel oder die Gruppierung

$>$NH steht,

Hetarylmethylamine der Formel (II)

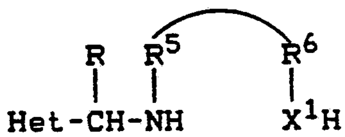

(II)

in welcher
Het, R, $R^5$, $R^6$ und $X^1$ die oben angegebene Bedeutung haben,
mit 1,1-Bismethylthio-4,4,4-trifluoro-1-buten-3-on der Formel (III)

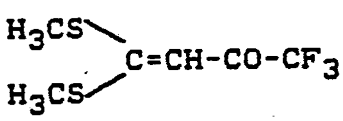

(III)

gegebenenfalls in Gegenwart von Verdünnungsmitteln umsetzt,
oder

(b) zum Erhalt von Verbindungen der Formel (Ib)

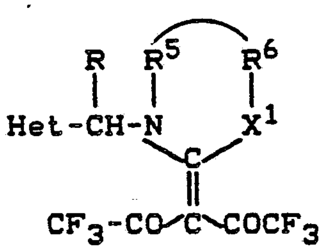

(Ib)

in welcher
Het, R, $R^5$, $R^6$ und $X^1$ die oben angegebene Bedeutung haben,
Trifluormethylcarbonyl-Derivative der Formel (Ia)

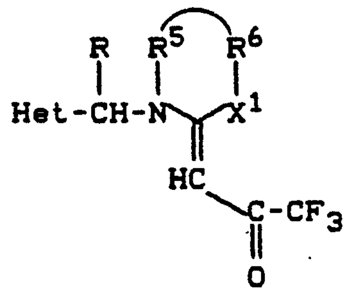

(Ia)

in welcher
Het, R, $R^5$, $R^6$ und $X^1$ die oben angegebenen Bedeutungen haben,
mit einem Trifluormethylcarbonsäure-Derivat der Formel (IV)

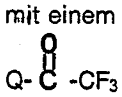 (IV)

in welcher
Q für Fluor, Chlor oder den Rest $-O-CO-CF_3$ steht,
gegebenenfalls in Gegenwart eines Säureakzeptors und gegebenenfalls in Gegenwart eines Verdünnungsmittels umsetzt,
oder

(c) zum Erhalt von Verbindungen der Formel (Ic)

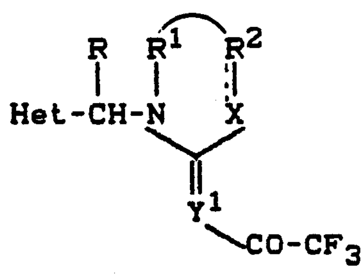

(Ic)

in welcher
Het, R, $R^1$, $R^2$, X die oben angegebene Bedeutung besitzen und

$Y^1$ für die Gruppierungen $>$CH oder $>$C-CO-$CF_3$ steht,
Verbindungen der Formel (V)

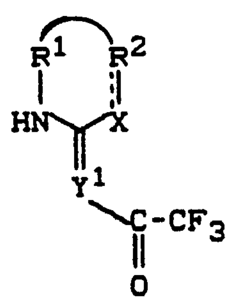

(V)

in welcher
$R^1$, $R^2$ und X die oben angegebenen Bedeutungen haben und
$Y^1$ für die Gruppierungen $>$CH oder $>$C-CO-$CF_3$ mit Halogeniden der Formel (VI)

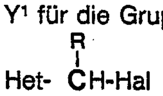 (VI)

in welcher
Het und R die oben angegebenen Bedeutungen haben und
Hal für Halogen steht,
gegebenenfalls in Gegenwart einer starken Base und gegebenenfalls in Gegenwart eines Verdünnungsmittels umsetzt,

oder

(d) zum Erhalt von Verbindungen der Formel (Id)

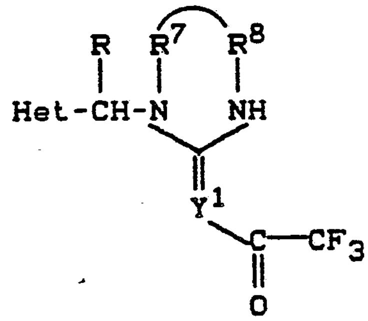

(Id)

in welcher

Het, R und $Y^1$ die oben angegebene Bedeutung haben und

$R^7$ und $R^8$ für einen gegebenenfalls durch $C_1$-$C_4$-Alkyl substituierten $C_2$-$C_3$-Alkylen-oder Alkenylen-Rest stehen,

Methyl-Derivate der Formel (VII)

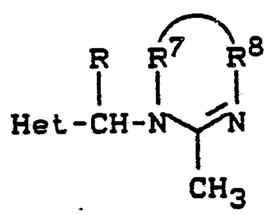

(VII)

in welcher

Het, R, $R^7$ und $R^8$ die oben angegebenen Bedeutungen haben,

($\alpha$) mit der zweifachen molaren Menge Trifluormethylcarbonsäure-Derivat der Formel (IV)

$$Q-\overset{O}{\overset{\|}{C}}-CF_3 \quad (IV)$$

in welcher

Q die oben angegebenen Bedeutungen hat,

gegebenenfalls in Gegenwart eines Säureakzeptors und gegebenenfalls in Gegenwart eines Verdünnungsmittels zu den Trifluormethylcarbonyl-Derivaten der Formel (Id-$\alpha$)

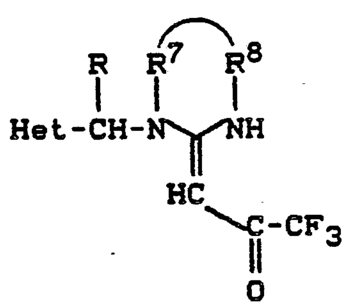

(Id-$\alpha$)

in welcher

Het, R, $R^7$ und $R^8$ die oben angegebene Bedeutungen haben,

umsetzt, oder

($\beta$) mit der dreifachen molaren Menge Trifluormethylcarbonsäure-Derivat der Formel (IV)

$$Q-\overset{O}{\overset{\|}{C}}-CF_3 \quad (IV)$$

in welcher

Q die oben angegebenen Bedeutungen hat,

gegebenenfalls in Gegenwart eines Säureakzeptors und gegebenenfalls in Gegenwart eines

Verdünnungsmittels zu den Trifluormethylcarbonyl-Derivaten der Formel (Id-β)

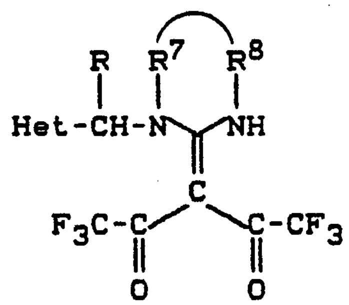

(Id-β)

in welcher
Het, R, $R^7$ und $R^8$ die oben angegebenen Bedeutungen haben,
umsetzt,
oder

(e) zum Erhalt von Verbindungen der Formel (Ie)

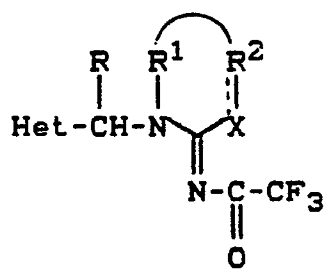

(Ie)

in welcher
Het, R, $R^1$, $R^2$, X die oben angegebene Bedeutung haben
Salze der Formel (VIII)

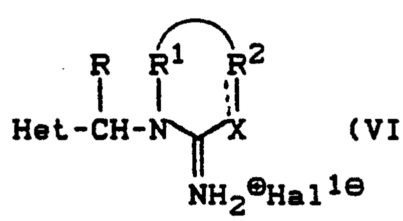

(VIII)

in welcher
Het, R, $R^1$, $R^2$ und X die oben angegebenen Bedeutungen haben und
$Hal^1$ für Halogen steht,
mit einem Trifluormethylcarbonsäure-Derivat der Formel (IV)

O
||
Q- C -$CF_3$ (IV)

in welcher
Q die oben angegebene Bedeutung hat,
gegebenenfalls in Gegenwart eines Säureakzeptors und gegebenenfalls in Gegenwart eines Verdünnungsmittels umsetzt.

Überraschenderweise zeichnen sich die erfindungsgemäßen Trifluormethylcarbonyl-Derivate der Formel (I), in überragender Weise durch eine hohe Wirksamkeit als Insektizide und Ektoparasitizide aus.

Die Erfindung betrifft vorzugsweise Verbindungen der Formel (I), in welcher
Het für gegebenenfalls durch Fluor, Chlor, Brom, Cyano, Nitro, $C_1$-$C_4$-Alkyl, $C_1$-$C_4$-Alkoxy, $C_1$-$C_4$-Alkylthio, Halogen-$C_1$-$C_4$-alkyl, Halogen-$C_1$-$C_4$-alkoxy, Halogen-$C_1$-$C_4$-alkylthio, Amino, $C_1$-$C_4$-Alkylamino oder Di-$C_1$-$C_4$-alkylamino substituiertes Furyl, Thiophenyl, Pyrazolyl, Imidazolyl, Pyrrolyl, 1,2,4-Triazolyl, 1,2,3-Triazolyl, Pryimidinyl, Pyrazinyl, Pyridyl, Oxazolyl, Isoxazolyl, 1,2,4-Oxadiazolyl, 1,3,4-Oxadiazolyl, Thiazolyl, Isothiazolyl, 1,2,5-Thiadiazolyl und 1,3,4-Thiaziazolyl steht,
R für Wasserstoff oder Methyl steht,
X für Sauerstoff, Schwefel, Stickstoff oder für die Gruppierungen

-NH und -CR$^4$ steht,
Y für Stickstoff oder den Rest -CR$^3$ steht, worin
R$^3$ für Wasserstoff oder eine Gruppierung -CO-CF$_3$ steht und
R$^1$ und R$^2$ gemeinsam mit dem angrenzenden Stickstoffatom und dem Rest X für einen heterocyclischen Rest der Formel

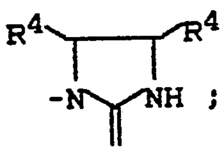 ; 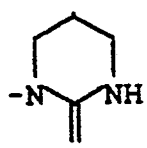 ; 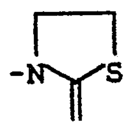 ; 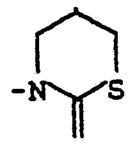 ;

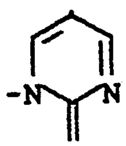 ; 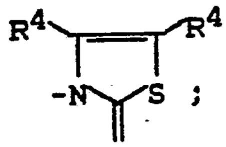 ; 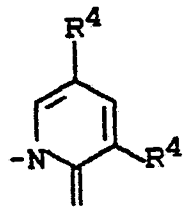 ; 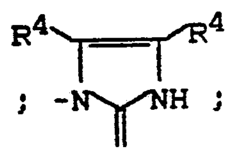 ;

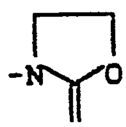 oder 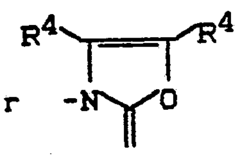

stehen, worin
R$^4$ für Wasserstoff oder $C_1$-$C_4$-Alkyl steht.

Besonders bevorzugt sind diejenigen Verbindungen der Formel (I), in welcher
Het für gegebenenfalls durch Fluor, Chlor, Brom, Cyano, Nitro, Methyl, Ethyl, Methoxy, Methylthio, Trifluormethyl, Trifluormethoxy, Trifluormethylthio, Dimethylamino oder Diethylamino substituiertes Thiophenyl, Pyrazolyl, 1,2,4-Triazolyl, Pyrazinyl, Pyrimidinyl, Pyridyl, Isoxazolyl, Oxazolyl, Thiazolyl, 1,2,5-Thiadiazolyl oder 1,3,4-Thiadiazolyl steht,
R für Wasserstoff steht,
X für Sauerstoff, Schwefel, Stickstoff oder für die Gruppierungen

-NH oder -CR$^4$

steht,
Y für Stickstoff oder den Rest

-CR$^3$

steht, worin
R$^3$ für Wasserstoff oder eine Gruppierung -CO-CF$_3$ steht und
R$^1$ und R$^2$ gemeinsam mit dem angrenzenden Stickstoffatom und dem Rest X für einen heterocyclischen Rest der Formel

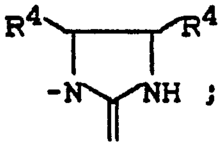 ; 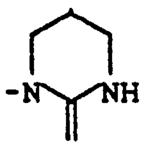 ; 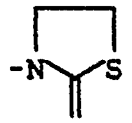 ; 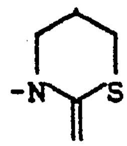 ;

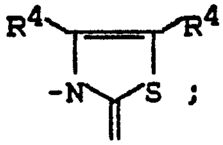 ; 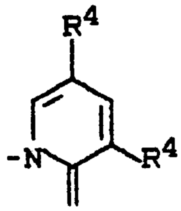 ; 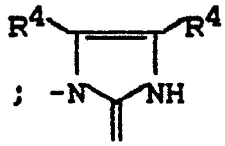

oder

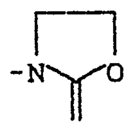

stehen, worin
R⁴ für Wasserstoff steht.

Verwendet man beispielsweise für das erfindungsgemäße Verfahren (a) 1,1-Bismethylthio-4,4,4-trifluor-1-buten-3-on und N-(3-Pyridyl-methyl)-N-(aminoethyl)-amin als Ausganstoffe, so kann die Reaktion durch das folgende Formelschema wiedergegeben werden:

(a)

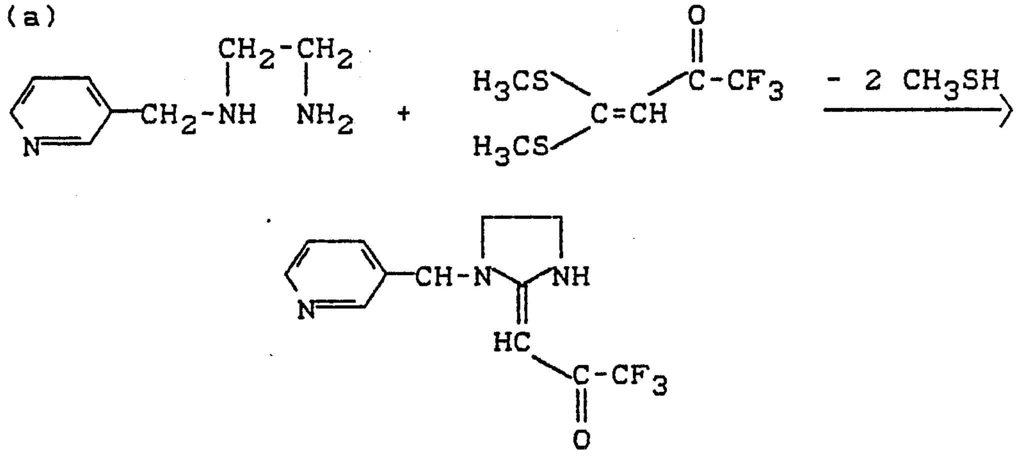


Verwendet man beispielsweise für das erfindungsgemäße Verfahren (b) 3-(6-Chlor-pyrid-3-yl)-methyl-2-(trifluormethylcarbonyl-vinyl)-1,3-thiazolidin und Trifluoressigsäureanhydrid als Ausgangsstoffe, so kann die Reaktion durch das folgende Formelschema wiedergegeben werden:

(b)

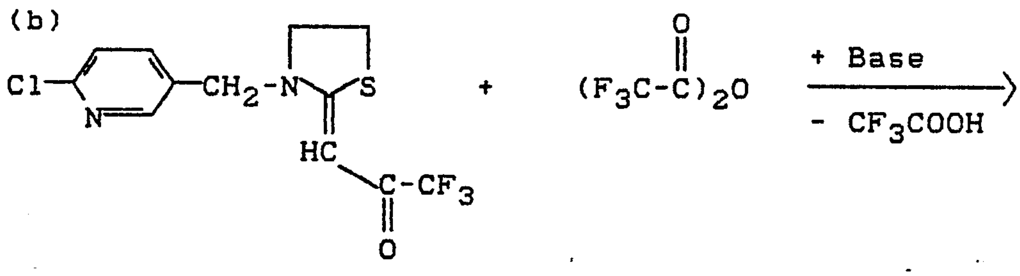

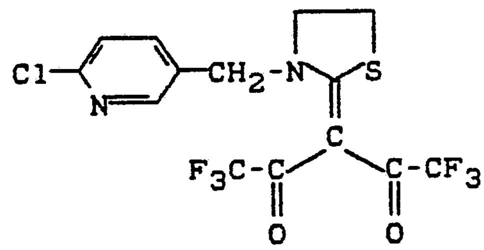

Verwendet man beispielsweise für das erfindungsgemäße Verfahren (c) 2-(Trifluormethylcarbonyl-vinyl)-1,3-thiazolidin und 2-Chlor-5-chlormethyl-pyridin als Ausgangsstoffe, so kann die Reaktion durch das folgende Formelschema wiedergegeben werden:

(c)

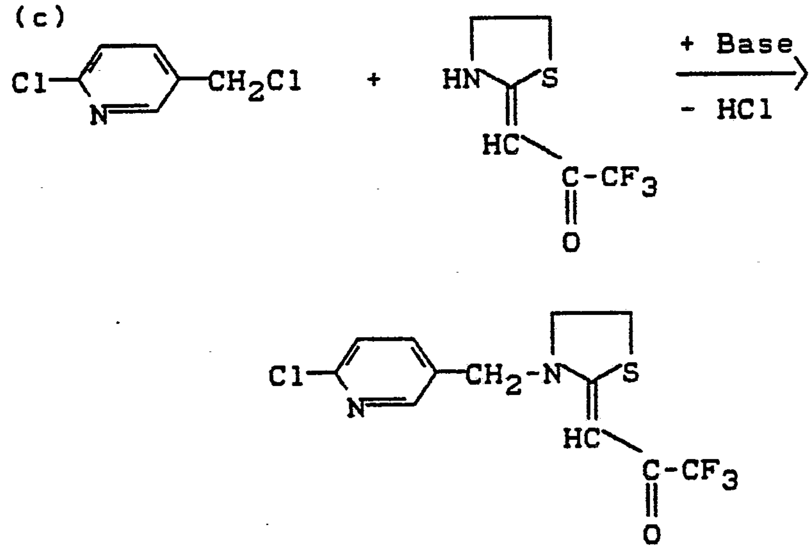


Verwendet man beispielsweise für das erfindungsgemäße Verfahren (d) als Ausgangsstoffe 1-(2-Chlor-pyrid-5-yl)-methyl-2-methyl-2-imidazolin und für die Verfahrensvariante (d/α) die zweifache molare Menge Trifluoressigsäureanhydrid bzw. für die Verfahrensvariante (d/β) die dreifache molare Menge Trifluoressigsäureanhydrid, so können die Reaktionen durch die folgenden Formelschemata wiedergegeben werden;

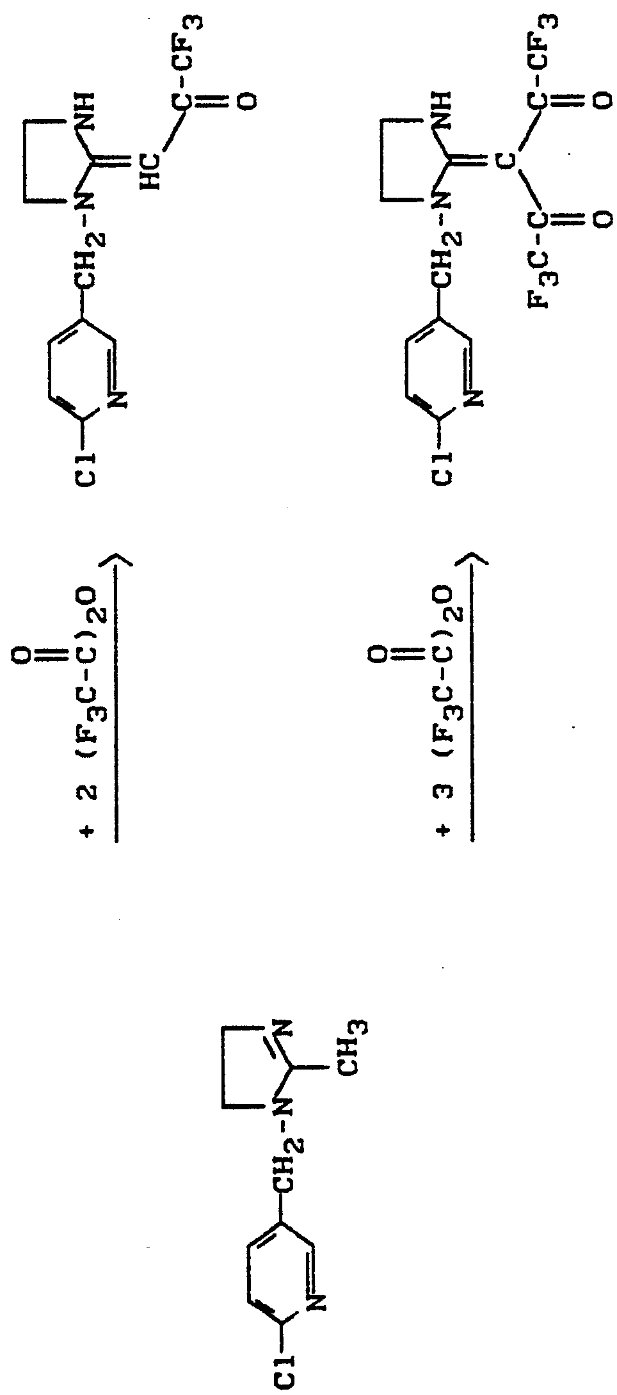

Verwendet man beispielsweise für das erfindungsgemäße Verfahren (e) 2-Amino-3-[2-chlor-pyrid-5-yl)-methyl]-thiazoliumchlorid und Trifluoressigsäureanhydrid als Ausgangsstoffe, so kann die Reaktion durch das folgende Formelschema wiedergegeben werden:

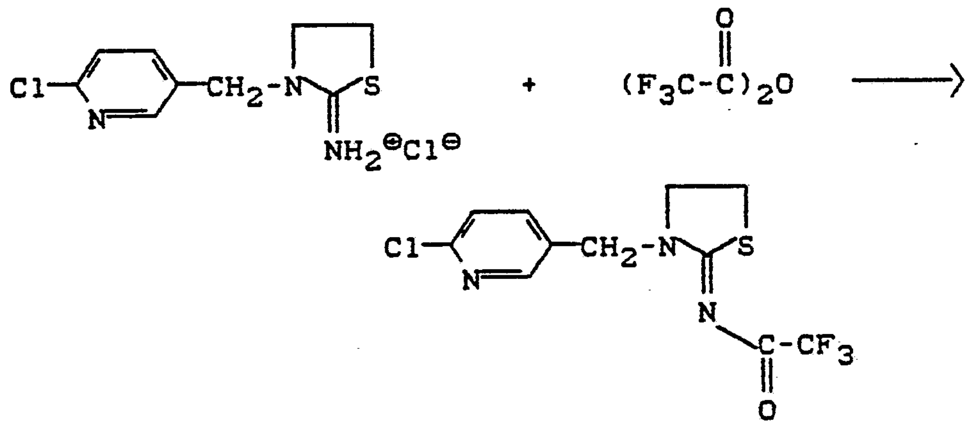

Die beim erfindungsgemäßen Verfahren (a) als Ausgangsstoffe zu verwendenden Hetarylmethylamine sind durch die Formel (II) allgemeine definiert. In dieser Formel (II) stehen Het und R vorzugsweise für diejenigen Reste, die bereits im Zusammenhant mit der Beschreibung der erfindungsgemäßen Stoffe der Formel (I) vorzugsweise für diese Substituenten genannt wurden. $R^5$ und $R^6$ stehen in dieser Formel gemeinsam für einen gegebenenfalls durch $C_1$-$C_4$-Alkyl substituierten $C_2$-$C_3$-Alkylen-Rest, vorzugsweise für einen gegebenenfalls durch Methyl substituierten $C_2$-$C_3$-Alkylen-Rest. $X^1$ steht in dieser Formel (II) für Sauerstoff, Schwefel oder die Gruppierung >NH.

Die Verbindungen der Formel (II) sind bekannt und/oder lassen sich nach bekannten Methoden herstellen (vgl. z. B. EP-A 154 178, EP-A 163 855 und EP-A 192 060).

Als Beispiele für die Hetarylmethylamine der Formel (II) seien genannt:

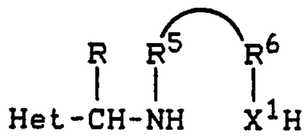 (II)

Tabelle 1

| Het | R | 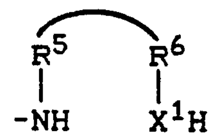 |
|---|---|---|
| 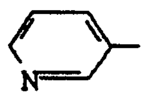 | H | $-NH-CH_2CH_2-NH_2$ |
| 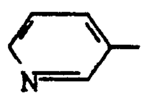 | H | $-NH-CH_2CH_2CH_2-NH_2$ |
| 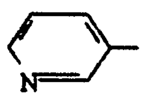 | $CH_3$ | $-NH-CH_2CH_2-NH_2$ |
| 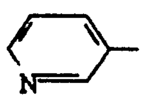 | H | $-NH-CH_2CH_2-SH$ |
| 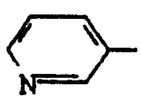 | H | $-NH-CH_2CH_2CH_2-SH$ |
| 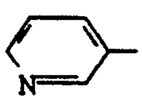 | H | $-NH-CH_2CH_2-OH$ |
| 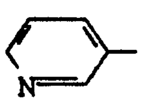 | H | $-NH-CH_2CH_2CH_2-OH$ |

<u>Tabelle 1</u> - Fortsetzung

| Het | R | 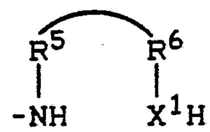 |
|---|---|---|
| 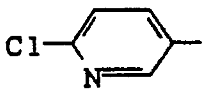 | H | $-NH-CH_2CH_2-NH_2$ |
| 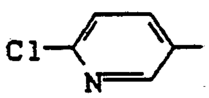 | H | 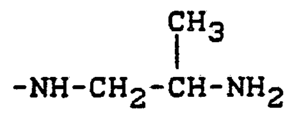 |
| 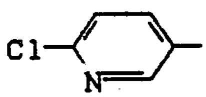 | H | $-NH-CH_2CH_2CH_2-NH_2$ |
| 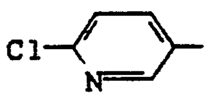 | $CH_3$ | $-NH-CH_2CH_2-NH_2$ |
| 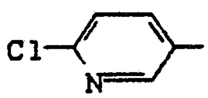 | H | $-NH-CH_2CH_2-SH$ |
| 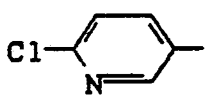 | H | $-NH-CH_2CH_2CH_2-SH$ |
| 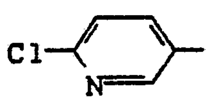 | H | $-NH-CH_2CH_2-OH$ |
| 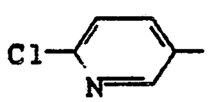 | H | $-NH-CH_2CH_2CH_2-OH$ |
| 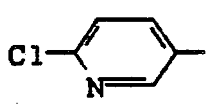 | $CH_3$ | $-NH-CH_2CH_2CH_2-NH_2$ |
| 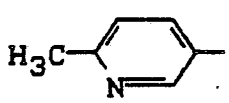 | H | $-NH-CH_2CH_2CH_2-SH$ |

Tabelle 1 - Fortsetzung

| Het | R | 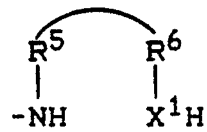 |
|---|---|---|
| 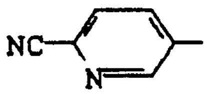 | H | $-NH-CH_2CH_2-NH_2$ |
| 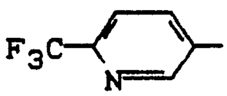 | H | $-NH-CH_2CH_2CH_2-NH_2$ |
| 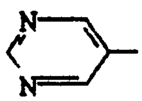 | H | $-NH-CH_2CH_2-NH_2$ |
| 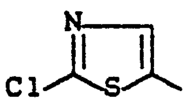 | H | $-NH-CH_2CH_2-NH_2$ |
| 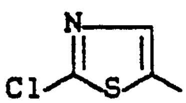 | H | $-NH-CH_2CH_2CH_2-NH_2$ |
| 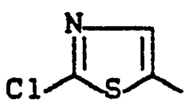 | H | $-NH-CH_2CH_2-OH$ |
| 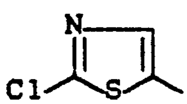 | H | $-NH-CH_2CH_2CH_2-OH$ |
| 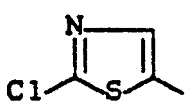 | H | $-NH-CH_2CH_2-SH$ |
| 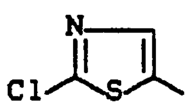 | H | $-NH-CH_2CH_2CH_2-SH$ |
| 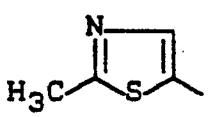 | H | $-NH-CH_2CH_2-NH_2$ |

<u>Tabelle 1</u> - Fortsetzung

| Het | R | 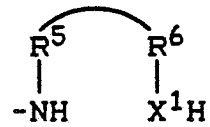 |
|---|---|---|
| 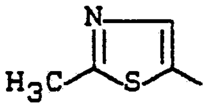 | H | $-NH-CH_2CH_2CH_2-NH_2$ |
| 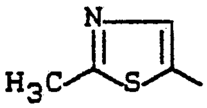 | H | $-NH-CH_2CH_2-OH$ |
| 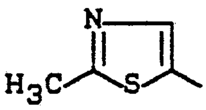 | H | $-NH-CH_2CH_2CH_2-OH$ |
| 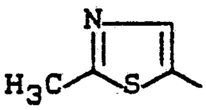 | H | $-NH-CH_2CH_2-SH$ |
| 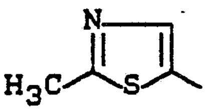 | H | $-NH-CH_2CH_2CH_2-SH$ |
| 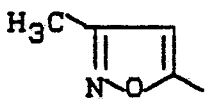 | H | $-NH-CH_2CH_2-NH_2$ |
| 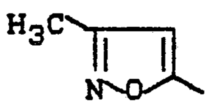 | H | $-NH-CH_2CH_2CH_2-NH_2$ |
| 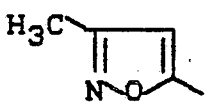 | H | $-NH-CH_2CH_2-OH$ |
| 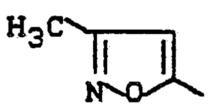 | H | $-NH-CH_2CH_2CH_2-OH$ |
| 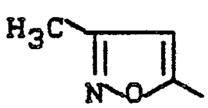 | H | $-NH-CH_2CH_2-SH$ |
| 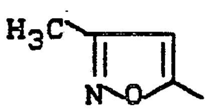 | H | $-NH-CH_2CH_2CH_2-SH$ |

Tabelle 1 - Fortsetzung

| Het | R | 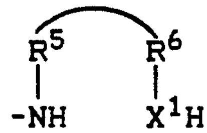 |
|---|---|---|
| 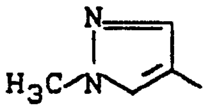 | H | $-NH-CH_2CH_2-NH_2$ |
| 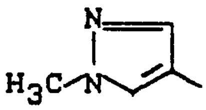 | H | $-NH-CH_2CH_2CH_2-NH_2$ |
| 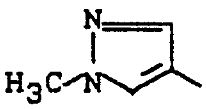 | H | $-NH-CH_2CH_2-OH$ |
| 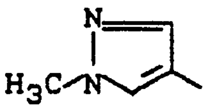 | H | $-NH-CH_2CH_2CH_2-OH$ |
| 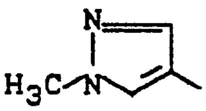 | H | $-NH-CH_2CH_2-SH$ |
| 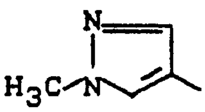 | H | $-NH-CH_2CH_2CH_2-SH$ |
| 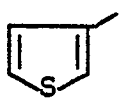 | H | $-NH-CH_2CH_2-NH_2$ |
| 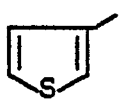 | H | $-NH-CH_2CH_2CH_2-NH_2$ |
| 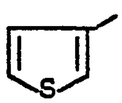 | H | $-NH-CH_2CH_2-OH$ |
| 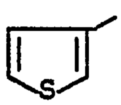 | H | $-NH-CH_2CH_2CH_2-OH$ |
| 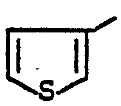 | H | $-NH-CH_2CH_2-SH$ |

Tabelle 1 - Fortsetzung

| Het | R | 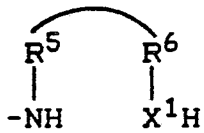 |
|---|---|---|
| 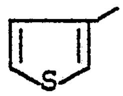 | H | $-NH-CH_2CH_2CH_2-SH$ |
| 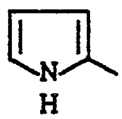 | H | $-NH-CH_2CH_2-NH_2$ |
| 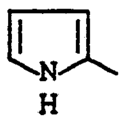 | H | $-NH-CH_2CH_2CH_2-NH_2$ |
| 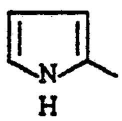 | H | $-NH-CH_2CH_2-OH$ |
| 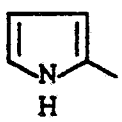 | H | $-NH-CH_2CH_2CH_2-OH$ |
| 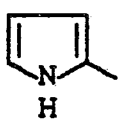 | H | $-NH-CH_2CH_2-SH$ |
| 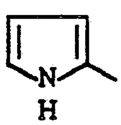 | H | $-NH-CH_2CH_2CH_2-SH$ |
| 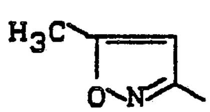 | H | $-NH-CH_2CH_2-NH_2$ |
| 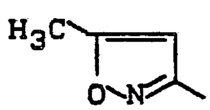 | H | $-NH-CH_2CH_2CH_2-NH_2$ |

Tabelle 1 - Fortsetzung

| Het | R | 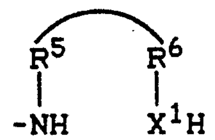 |
|---|---|---|
| 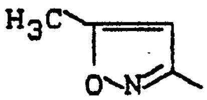 | H | $-NH-CH_2CH_2-OH$ |
| 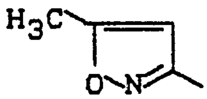 | H | $-NH-CH_2CH_2CH_2-OH$ |
| 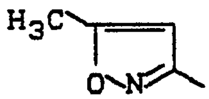 | H | $-NH-CH_2CH_2-SH$ |
| 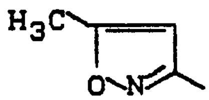 | H | $-NH-CH_2CH_2CH_2-SH$ |
| 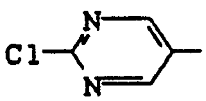 | H | $-NH-CH_2CH_2-NH_2$ |
| 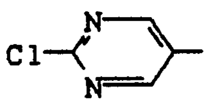 | H | $-NH-CH_2CH_2CH_2-NH_2$ |
| 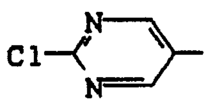 | H | $-NH-CH_2CH_2-OH$ |
| 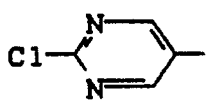 | H | $-NH-CH_2CH_2CH_2-OH$ |
| 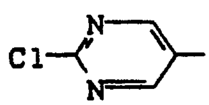 | H | $-NH-CH_2CH_2-SH$ |
| 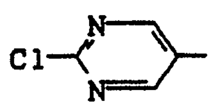 | H | $-NH-CH_2CH_2CH_2-SH$ |

Die weiterhin als Ausgangstoff für das erfindungsgemäße Verfahren (a) zu verwendende Verbindung 1,1-Bismethylthio-4,4,4-trifluor-1-buten-3-on der Formel (III) ist neu und Teil der vorliegenden Erfindung. Sie läßt sich auf einfache Weise herstellen, indem man 1,1,1-Trifluoraceton in Gegenwart von starken Basen, wie z. B. Natriumhydrid und in Gegenwart von inerten Lösungsmitteln wie z. B. Tetrahydrofuran, Dimethylformamid oder Dimethylsulfoxid mit Schwefelkohlenstoff und einem Methylierungsmittel wie z. B. Methyliodid bei Temperaturen zwischen 0 °C und 60 °C umsetzt. Die Aufarbeitung des Reaktionsproduktes geschieht nach üblichen Methoden.

Das erfindungsgemäße Verfahren (a) zur Herstellung der neuen Trifluormethylcarbonyl-Derivate der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogeniert Kohlenwasserstoffe wie

Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran and Dioxan, Alkohole wie Methanol, Ethanol, n-Propanol, i-Propanol und n-Butanol, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Die Reaktionstemperaturen können bei dem erfindungsgemäsen Verfahren (a) in einem größeren Bereich variiert werden. Im allgemeinen arbeitet man bei Temperaturen zwischen 0 °C und 180 °C, vorzugsweise bei Temperaturen zwischen 20 °C und 160 °C. Das erfindungsgemäße Verfahren (a) wird im allgemeinen unter Normaldruck durchgeführt.

Zur Durchführung des erfindungsgemäßen Verfahrens (a) werden die jeweils benötigten Ausgangsstoffe im allgemeinen in angenähert äquimolaren Mengen eingesetzt. Die Reaktionen werden im allgemeinen in einem geeigneten Verdünnungsmittel durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden.

Die beim erfindungsgemäßen Verfahren (b) als Ausgangsstoffe zu verwendenden Trifluormethylcarbonyl-Derivate sind durch die Formel (Ia) allgemein definiert. In dieser Formel (Ia) stehen Het, $R^1$ und $R^2$ vorzugsweise für diejenigen Reste, die bereits im Zusammenhang mit der Beschreibung der erfindungsgemäßen Stoffe der Formel (I) vorzugsweise für diese Substituenten genannt wurden. $X^1$ steht in dieser Formel für Sauerstoff, Schwefel oder die Gruppierung >NH.

Die Verbindungen der Formel (Ia) sind erfindungsgemäße Verbindungen und lassen sich nach den erfindungsgemäßen Verfahren (a), (c) und (d/α) herstellen.

Als Beispiele für die Verbindungen der Formel (Ia) seien genannt:

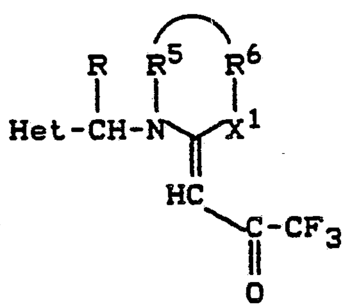

(Ia)

R = H, $CH_3$

Tabelle 2

| Het | 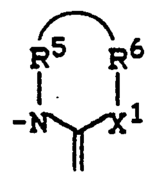 | Het | 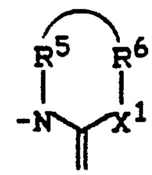 |
|---|---|---|---|
| 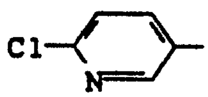 | 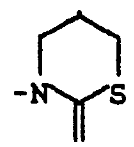 | 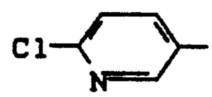 | 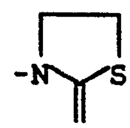 |
| 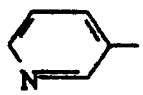 | 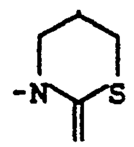 | 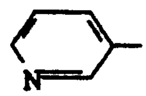 | 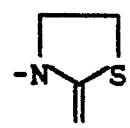 |
| 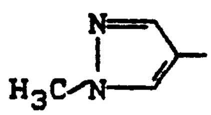 | 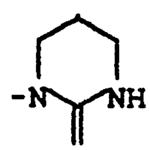 | 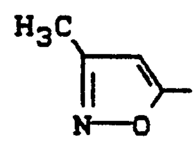 | 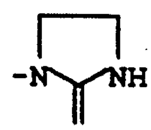 |
| 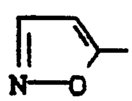 | 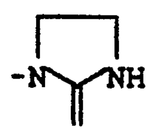 | 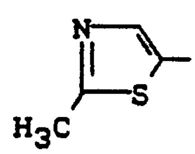 | 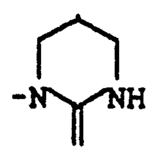 |
| 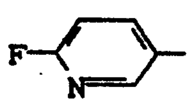 | 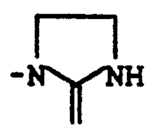 | 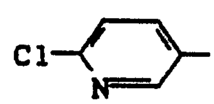 | 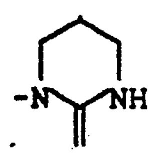 |

Tabelle 2 - Fortsetzung

| Het | 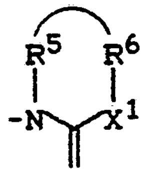 | Het | 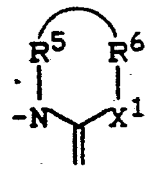 |
|---|---|---|---|
| 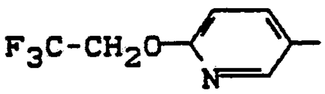 | 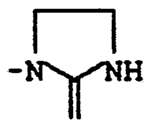 | 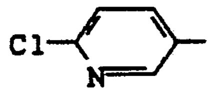 | 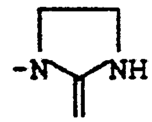 |
| 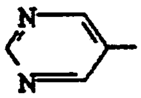 | 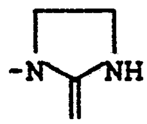 | 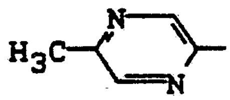 | 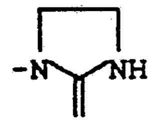 |
| 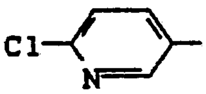 | 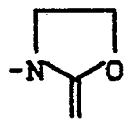 | 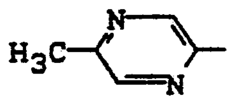 | 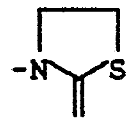 |
| 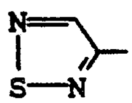 | 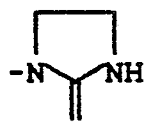 | 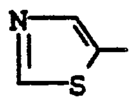 | 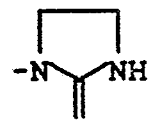 |
| 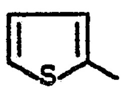 | 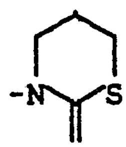 | 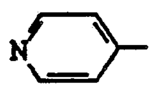 | 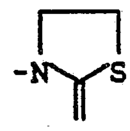 |
| 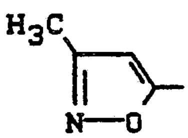 | 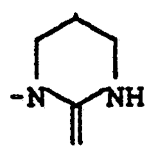 | 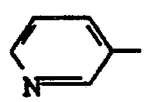 | 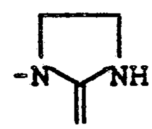 |
| 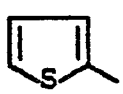 | 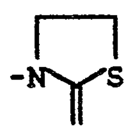 | 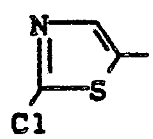 | 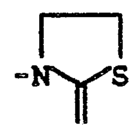 |

Tabelle 2 - Fortsetzung

| Het | 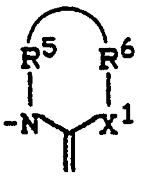 | Het | 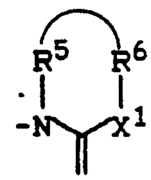 |
| --- | --- | --- | --- |
| 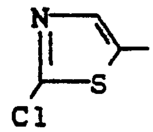 | 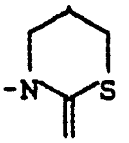 | 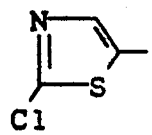 | 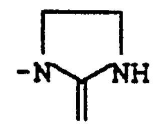 |
| 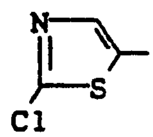 | 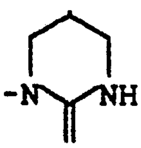 | 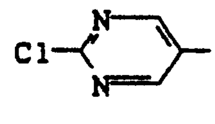 | 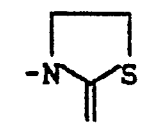 |
| 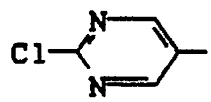 | 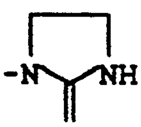 | 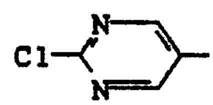 | 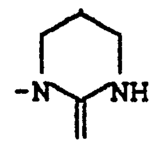 |

Die bei dem erfindungsgemäßen Verfahren (b) außerdem als Ausgangsstoffe zu verwendenden Trifluormethylcarbonsäure-Derivate sind durch die Formel (IV) allgemein definiert. In dieser Formel (IV) steht Q für Fluor, Chlor oder den Rest, $-O-CO-CF_3$. Vorzugsweise steht Q für $-O-CO-CF_3$.

Als Beispiele für die Verbindung der Formel (IV) seien genant:

1,1,1-Trifluoressigsäure-anhydrid, -fluorid und -chlorid.

Das erfindungsgemäße Verfahren (b) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Nitrile wie z. B. Acetonitril und Propionitril.

Als Säureakzeptoren können bei dem erfindungsgemäßen Verfahren alle üblicherweise für derartige Umsetzungen verwendbaren Säurebindemittel eingesetzt werden. Vorzugsweise in Frage kommen Alkalihydrogencarbonate wie z. B. Natrium-und Kaliumhydrogencarbonat, Alkalicarbonate und Erdalkalicarbonate wie Natrium-, Kalium-und Calciumcarbonate, ferner aliphatsiche, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo[2,2,2]-octan (DABCO).

Die Reaktionstemperaturen können bei dem erfindungsmäßen Verfahren (b) in einem größeren Bereich variiert werden. Im allgemeinen arbeitet man bei Temperaturen zwischen 0 °C und 60 °C, vorzugsweise bei Temperaturen zwischen 0 °C und 40 °C. Das erfindungsgemäße Verfahren (b) wird im allgemeinen unter Normaldruck durchgeführt.

Bei der Durchführung des erfindungsgemäßen Verfahrens (b) setzt man auf 1 Mol der Verbindung der Formel (Ia) 2 bis 3, vorzugsweise 2 bis 2,2 Mol Trifluormethylcarbonsäure-Derivat der Formel (IV) ein. Die Reaktionen werden im allgemeinen in einen geeigneten Verdünnungsmittel und in Gegenwart eines Säureakzeptors durchgeführt und das Reaktionsgemisch wird mehrere Stunden bei der jeweils erforderlichen Temperatur gerührt. Die Aufarbeitung geschieht nach üblichen Methoden.

Die beim erfindungsgemäßen Verfahren (c) als Ausgangsstoffe zu verwendenden Verbindungen sind durch die Formel (V) allgemein definiert. In dieser Formel (V) stehen $R^1$, $R^2$ und X vorzugsweise für diejenigen Reste, die bereits im Zusammenhang mit der Beschreibung der erfindungsgemäßen Stoffe der Formel (I) vorzugsweise für diese Substituenten genannt wurden. $Y^1$ steht in dieser Formel für die Gruppierungen $>CH$ oder $>C-CO-CF_3$.

Als Beispiele für die Verbindungen der Formel (V) seien genannt:

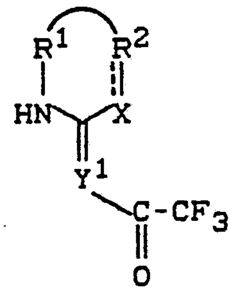

(V)

Tabelle 3

| 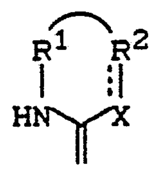 | $Y^1$ | 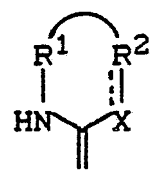 | $Y^1$ |
|---|---|---|---|
| 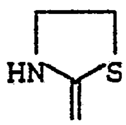 | CH | 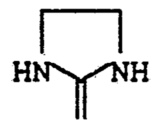 | CH |
| 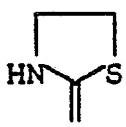 | $C-COCF_3$ | 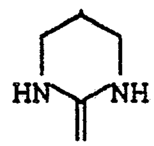 | CH |
| 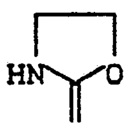 | CH | 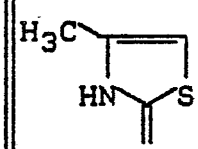 | CH |
| 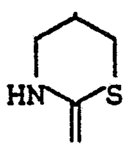 | CH | 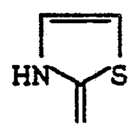 | CH |
| 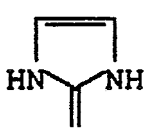 | $C-COCF_3$ | 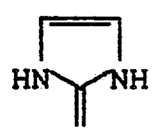 | CH |

Die Verbindungen der Formel (V) sind zum Teil bekannt (vgl. US-PS 45 51 447). Neu und Teil der vorliegenden Erfindung sind die Verbindungen der Formel (Va)

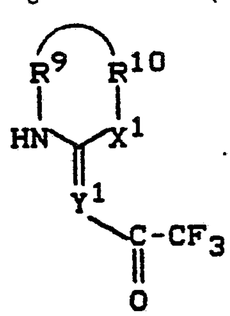

(Va)

in welcher

$Y^1$ für die Gruppierungen $>CH$ oder $>C-CO-CF_3$ steht, X für Sauerstoff, Schwefel oder für die Gruppierung $>NH$ steht und

$R^9$ und $R^{10}$ gemeinsam mit dem angrenzenden Stickstoffatom und den Rest $X^1$ für einen Heterocyclus der Formel

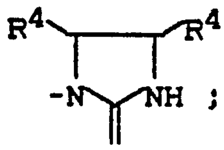 ; 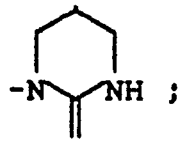 ; 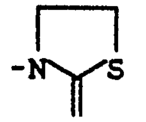 ; 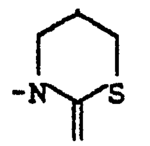 ;

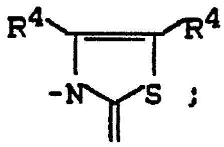 ; 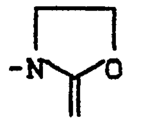 oder 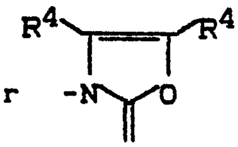

stehen, worin
$R^4$ für Wasserstoff oder Alkyl steht.

Die neuen Verbindungen der Formel (Va) lassen sich nach bekannten Methoden herstellen, z. B. indem man Verbindungen der Formel (IX)

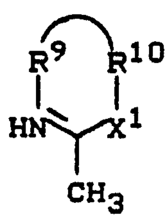 (IX)

in welcher
$R^9$, $R^{10}$ und $X^1$ die oben angegebenen Bedeutungen haben,
mit Trifluormethylcarbonsäure-Derivaten der Formel (IV)

$Q-\overset{O}{\overset{\|}{C}}-CF_3$ (IV)

in welcher
Q die oben angegebenen Bedeutungen hat,
in Gegenwart von Säureakzeptoren, wie z. B. Triethylamin oder Pyridin und in Gegenwart von inerten Lösungsmitteln, wie z. B. Dichlormethan oder Acetonitril bei Temperaturen zwischen -10 °C und +40 °C umsetzt.

Die für die Herstellung von den Verbindungen der Formel (Va) zu verwendenden Ausgangsprodukte der Formel (IX) sind bekannte Verbindungen der organischen Chemie (vgl. z. B. J. Org. Chem. 40, 252 (1975)).

Die beim erfindungsgemäßen Verfahren (c) außerdem als Ausgangsstoffe zu verwendenden Halogenide sind durch die Formel (VI) allgmein definiert. In dieser Formel (VI) stehen Het und R vorzugsweise für diejenigen Reste, die bereits im Zusammenhang mit der Beschreibung der erfindungsgemäßen Stoffe der Formel (I) vorzugsweise für diese Substituenten genannt wurden. Hal steht in dieser Formel für Halogen, vorzugsweise für Chlor oder Brom.

Die Verbindungen der Formel (VI) sind bekannt oder lassen sich nach bekannten Verfahren herstellen (vgl. z. B. EP-A 192 060; EP-A 163 855; EP-A 154 178; J. Org. Chem. 34, 3547; J. Med. Chem. 14, 211 - 213 und 557 -558 (1971); US-PS 4 332 944 und J. Het. Chem. 1979, 333 - 337).

Als Beispiele für die Verbindungen der Formel (VI) seien genannt:

$Het-\overset{R}{\overset{|}{C}}H-Hal$ (VI)

Hal = Cl oder Br.

Tabelle 4

| Het | R | Het | R |
|---|---|---|---|
| 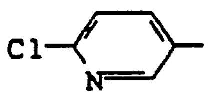 | H | 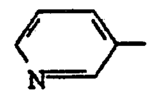 | H |
| 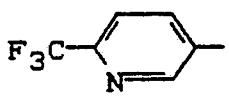 | H | 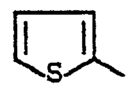 | H |
| 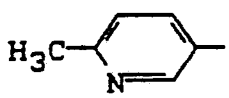 | H | 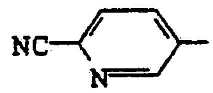 | H |
| 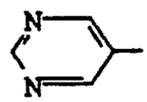 | H | 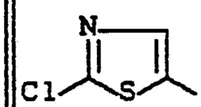 | H |
| 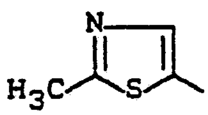 | H | 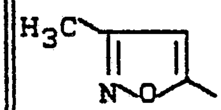 | H |
| 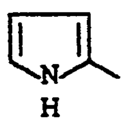 | H | 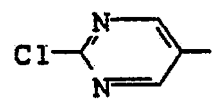 | H |
| 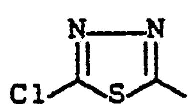 | H | | |

Das erfindungsgemäße Verfahren (c) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise Ether wie Diethyl-und Diethylether, Glykoldimethylether und Diglykoldimethylether, Tetrahydrofuran und Dioxan, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (c) alle üblicherweise für derartiger Umsetzungen verwendbaren Säurebindemittel eingesetzt werden. Vorzugsweise in Frage kommen Alkalimetallhydride wie z. B. Natrium-und Kaliumhydrid, Alkalicarbonate und -alkoholate wie Natrium-und Kaliumcarbonat, Natrium-und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (c) in einem größeren Bereich variiert werden. Im allgemeinen arbeitet man bei Temperaturen zwischen 0 °C und 160 °C, vorzugsweise bei Temperaturen zwischen 20 °C und 120 °C. Das erfindungsgemäße Verfahren (c) wird im allgemeinen unter Normaldruck durchgeführt.

Zur Durchführung des erfindungsgemäßen Verfahrens (c) werden die jeweils benötigten Ausgangsstoffe und der Säureakzeptor im allgemeinen in angenähert äquimolaren Mengen eingesetzt. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (c) jeweils nach üblichen Methoden.

Die beim erfindungsgemäßen Verfahren (d) als Ausgangsstoffe zu verwendenden Methyl-Derivate sind durch die Formel (VII) allgemein definiert. In dieser Formel (VII) stehen Het und R vorzugsweise für diejenigen Reste, die bereits im Zusammenhang mit der Beschreibung der erfindungsgemäßen Stoffe der Formel (I) vorzugsweise für diese Substituenten genannt wurden, $R^7$ und $R^8$ stehen in dieser Formel gemeinsam für einen gegebenenfalls durch $C_1$-$C_4$-Alkyl substituierten $C_2$-$C_3$-Alkylen-oder Alkenylen-Rest, vorzugsweise für einen gegebenenfalls durch Methyl substituierten $C_2$-$C_3$-Alkylen-oder Alkenylen-Rest stehen.

Als Beispiele für die Methyl-Derivate der Formel (VII) seien genannt:

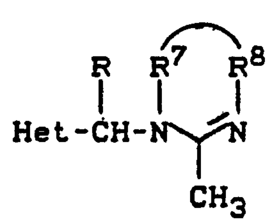

(VII)

Tabelle 5

| Het | R | 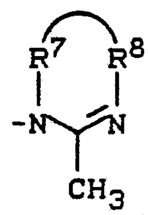 |
|---|---|---|
| 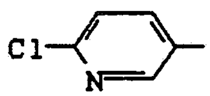 | H | 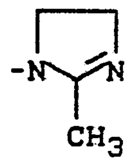 |
| 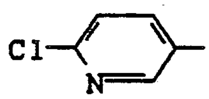 | H | 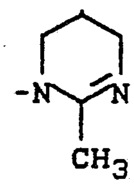 |
| 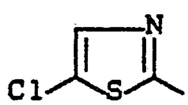 | H | 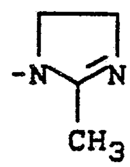 |
| 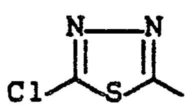 | H | 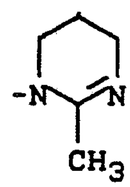 |
| 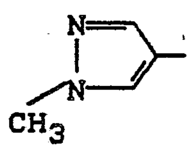 | H | 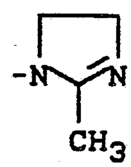 |
| 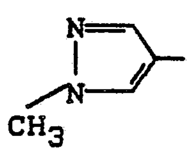 | H | 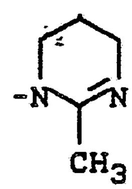 |

Tabelle 5 - Fortsetzung

| Het | R | 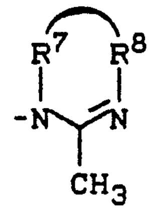 |
|---|---|---|
| 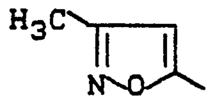 | H | 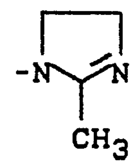 |
| 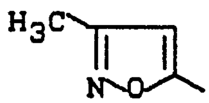 | H | 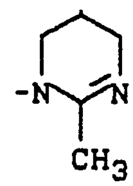 |
| 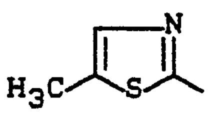 | H | 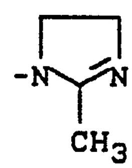 |
| 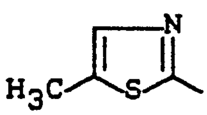 | H | 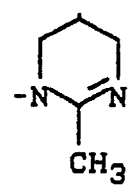 |
| 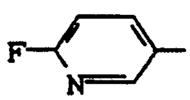 | H | 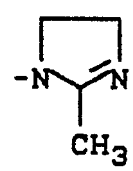 |
| 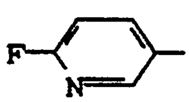 | H | 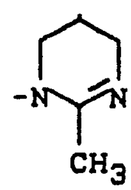 |

Die Verbindungen der Formel (VII) sind neu und Teil der vorliegenden Erfindung. Sie lassen sich auf einfache Weise herstellen, indem man Hetarylmethylamine der Formel (IIa)

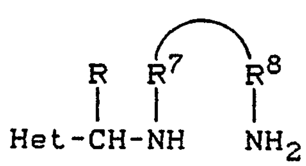 (IIa)

in welcher

Het, R, $R^7$ und $R^8$ die oben angegebenen Bedeutungen haben,

mit Orthoessigsäureestern der Formel (X)

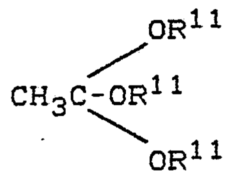

(X)

in welcher
$R^{11}$ für $C_1$-$C_4$-Alkyl steht,
gegebenenfalls in Gegenwart von Katalysatoren, wie z. B. p-Toluolsulfonsäure und gegebenenfalls in Gegenwart von inerten Verdünnungsmittel wie z. B. Xylol oder Toluol bei Temperaturen zwischen 20 °C und 160 °C umsetzt.

Die Verbindungen der Formel (IIa) wurden bereits bei der Beschreibung der Verbindungen der Formel (II) ($X^1$ = >NH) beschrieben.

Die Orthoessigsäureester der Formel (X) sind bekannte Verbindungen der organischen Chemie.

Als Beispiele für die Verbindungen der Formel (X) seien beispielsweise genannt:
Orthoessigsäureester-trimethylester, -triethylester und -tri-n-propylester.

Die weiterhin als Ausgangsstoffe für das erfindungsgemäße Verfahren (d, Variante $\alpha$ und Variante $\beta$) zu verwendenden Trifluormethylcarbonyl-Derivate der Formel (IV) wurden bereits bei der Beschreibung des Verfahrens (b) beschrieben.

Für die Durchführung des erfindungsgemäßen Verfahrens (d/Variante $\alpha$ und $\beta$) kommen diejenigen Verdünnungsmitel und Säureakzeptoren in Frage, die bereits bei der Beschreibung des erfindungsgemäßen Verfahrens (b) angegeben wurden.

Die Reaktionstemperaturen können bei dem erfindungsgemäßen Verfahren (d) für beide Varianten ($\alpha$) und ($\beta$) in einem größeren Bereich variiert werden. Im allgemeinen arbeitet man bei Temperaturen zwischen 0 °C und 60 °C, vorzugsweise bei Temperaturen zwischen 0 °C und 40 °C. Das erfindungsgemäße Verfahren (d/$\alpha$ und d/$\beta$) wird im allgemeinen unter Normaldruck durchgeführt.

Bei der Durchführung des erfindungsgemäßen Verfahrens (d) setzt man auf 1 Mol der Verbindung der Formel (VII), gemäß Variante (d/$\alpha$) 2 Mol der Verbindung der Formel (IV), für den Fall, daß Verbindungen der Formel (Id-$\alpha$) hergestellt werden sollen. Für den Fall, daß Verbindungen der Formel (Id-$\beta$) hergestellt werden sollen, setzt man gemäß Variante (d/$\beta$) 3 Mol der Verbindung der Formel (IV) auf 1 Mol der Verbindung der Formel (VII) ein. Die Aufarbeitung der Reaktionsprodukte geschieht nach üblichen Methoden.

Die beim erfindungsgemäßen Verfahren (e) als Ausgangsstoffe zu verwendenden Salze sind durch die Formel (VIII) allgemein definiert. In dieser Formel (VIII) stehen Het, R, $R^1$ und $R^2$ vorzugsweise für diejenigen Reste, die bereits im Zusammenhang mit der Beschreibung der erfindungsgemäßen Stoffe der Formel (I) vorzugsweise für diese Substituenten genannt wurden. $Hal^1$ steht in dieser Formel für Halogen, vorzugsweise für Chlor oder Brom.

Die Verbindungen der Formel (VIII) sind bekannt und/oder lassen sich nach bekannten Methoden herstellen (vgl. CA. 61, 652 a - e; Chem. Berichte 54, 814 (1921) und 88, 1103 (1955) und J. Chem. Soc. 1939, 1855 und Herstellungsbeispiele).

Als Beispiele für die Salze der Formel (VIII) seien genannt:

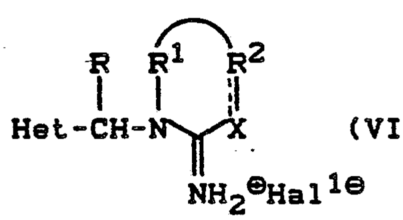

(VIII)

Hal = Cl oder Br
R = H oder $CH_3$

Tabelle 6

| Het | 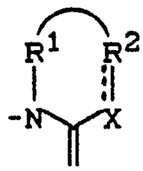 | Het | 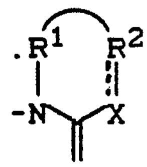 |
|---|---|---|---|
| 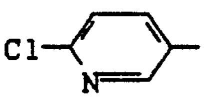 | 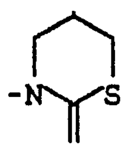 | 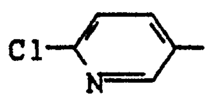 | 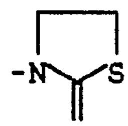 |
| 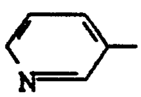 | 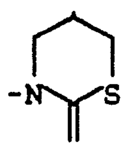 | 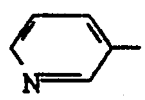 | 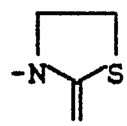 |
| 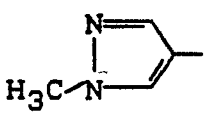 | 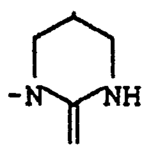 | 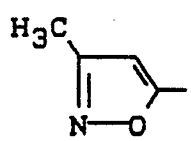 | 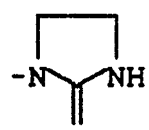 |
| 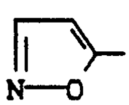 | 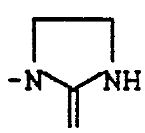 | 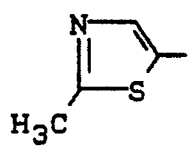 | 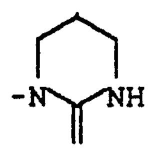 |
| 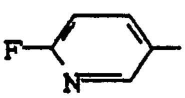 | 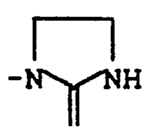 | 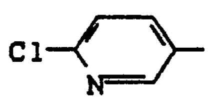 | 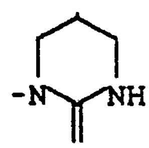 |
| 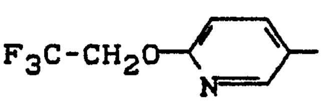 | 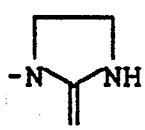 | 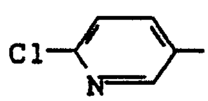 | 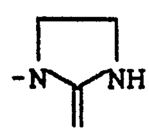 |
| 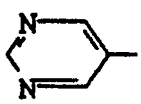 | 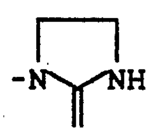 | 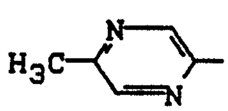 | 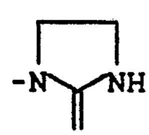 |
| 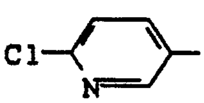 | 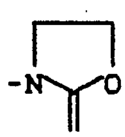 | 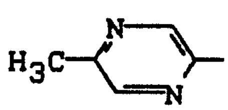 | 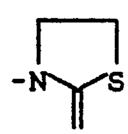 |

Tabelle 6 - Fortsetzung

| Het | 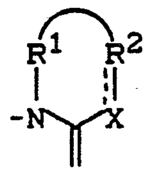 | Het | 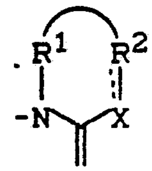 |
|---|---|---|---|
| 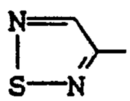 | 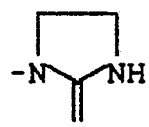 | 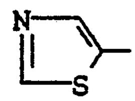 | 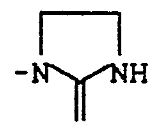 |
| 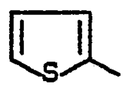 | 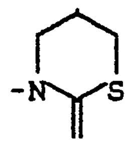 | 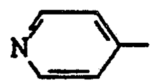 | 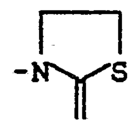 |
| 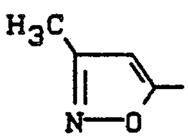 | 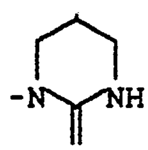 | 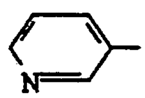 | 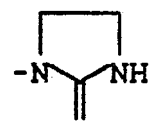 |
| 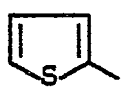 | 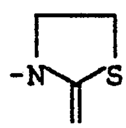 | 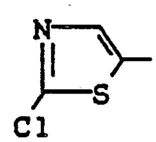 | 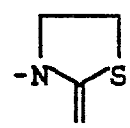 |
| 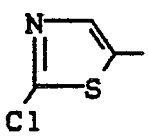 | 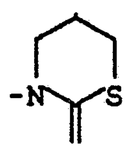 | 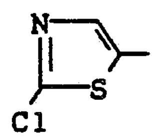 | 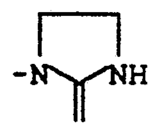 |
| 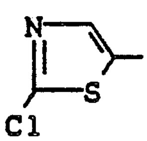 | 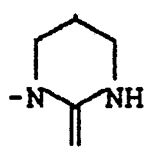 | 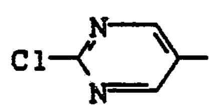 | 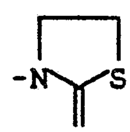 |
| 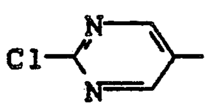 | 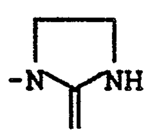 | 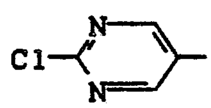 | 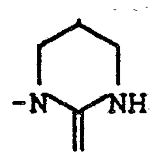 |
| 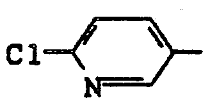 | 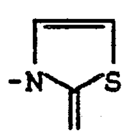 | 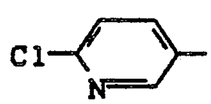 | 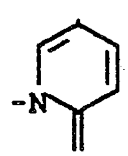 |

Tabelle 6 - Fortsetzung

| Het | 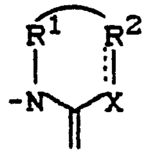 | Het | 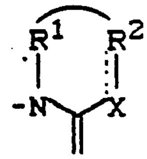 |
|---|---|---|---|
| 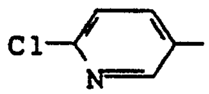 | 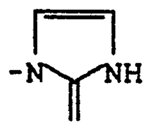 | 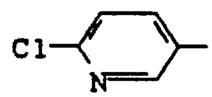 | 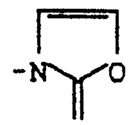 |
| 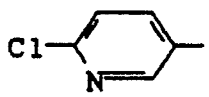 | 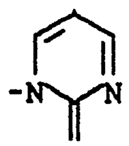 | 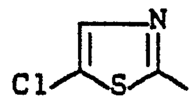 | 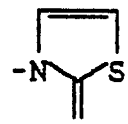 |
| 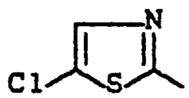 | 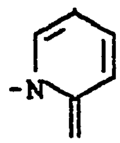 | 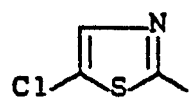 | 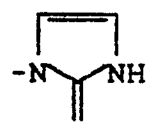 |
| 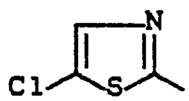 | 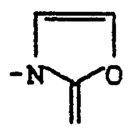 | 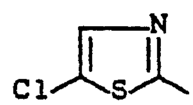 | 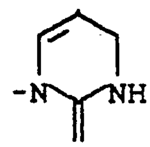 |
| | | 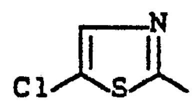 | 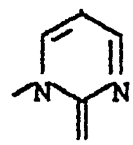 |

Die weiterhin als Ausgangsstoffe für das erfindungsgemäße Verfahren (e) zu verwendende Trifluormethylcarbonyl-Derivate der Formel (IV) wurden bereits bei der Beschreibung des Verfahrens (b) beschrieben.

Das erfindungsgemäße Verfahren (e) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol und Nitrile wie z. B. Acetonitril und Propionitril.

Als Säureakzeptoren können bei dem erfindungsgemäßen Verfahren (e) alle üblicherweise für derartige Umsetzungen verwendbaren Säurebindemittel eingesetzt werden. Vorzugsweise in Frage kommen Alkalimetallhydroxide wie z. B. Natrium-und Kaliumhydroxid, Erdalkalihydroxide wie z. B. Calciumhydroxid, Alkalicarbonate und -alkoholate wie Natrium-und Kaliumcarbonat, Natrium-und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (e) in einem größeren Bereich variiert werden. Im allgemeinen arbeitet man bei Temperaturen zwischen 0 °C und 100 °C, vorzugsweise bei Temperaturen zwischen 20 °C und 80 °C.

Das erfindungsgemäße Verfahren (e) wird im allgemeinen unter Normaldruck durchgeführt. Es ist jedoch auch möglich, unter erhöhtem oder vermindertem Druck zu arbeiten.

Bei der Durchführung des erfindungsgemäßen Verfahrens (e) setzt man auf 1 Mol der Verbindung der Formel (VIII) 1 Mol bis 1,5 Mol, vorzugsweise 1 Mol bis 1,2 Mol der Verbindung der Formel (IV) ein. Die Aufarbeitung der Reaktionsprodukte geschieht nach üblichen Methoden.

Die Wirkstoffe eignen sich bei guter Pflanzenverträglichkeit und günstiger Warmblütertoxizität zur Bekämpfung von tierischen Schädlingen, insbesondere Insekten, die in der Landwirtschaft, in Forsten, im Vorrats-und Materialschutz sowie auf dem Hygienesektor, vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ornung der Orthoptera z.B. Blatta orientalis.

Periplaneta americana, Leucophaea maderae, Blattella germanica, Acehta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium cornis, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallen solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Die erfindungsgemäßen Wirkstoffe der Formel (I) zeichnen sich durch eine hervorragende insektizide Wirksamkeit aus. Sie zeigen insbesondere beim Einsatz als Blattinsektizide und Bodeninsektizide eine hervorragende Wirkung gegen Zikaden, wie z. B. Nephotettix cincticeps, gegen Käferlarven, wie z.B. Phaedon cochleariae und Blattläuse, wie z.B. Myzus persicae und Aphis fabae.

Die neuen Verbindungen sind also besonders gut für einen Einsatz zur Bekämpfung von Blattinsekten und Bodeninsekten geeignet.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage; z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage; z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägmehl, Kokosnußschalen, Maiskolben und Tabakstengel; als Emulgier und/oder shcaumerzeugende Mittel kommen in Frage; z.B. nichtionogene und anionische Emulgatoren, wie Polyoxyethylen-Fettsäure-Ester, Polyoxy ethylen-Fettalkohol-Ether, z.B. Alkylarylpolyglykol-Ether, Alkylsulfonate, Alkysulfate, Arylsulfonate sowie Eiweißhydrolysate; als Dispergiermittel kommen in Frage; z.B. Lignin-Sulfitablaugen und Methylcellulose.

Es können in den Formulierungen Haftmittel wie Carboxymethylcellulose, näturliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäure ester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene-und Vorratsschädlinge zeichnen sich die Wirkstoffe durch eine hervorragende Residualwirkung auf Holz und Ton sowie durch eine gute Alkalistabilität auf gekälten Unterlagen aus.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hüh ner, Puten, Enten, Gänse, sonstige Haustiere wie z.B. Hunde, Katzen, Stubenvögel, Aquarienfische sowie sog. Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmäßenbändern, Halftern, Markierungsvorrichtungen usw.

Sie zeigen insbesondere beim Einsatz als Ektoparasitizide eine ausgezeichnete Wirkung gegen Blowflylarven wie z.B. Lucilia cuprina.

Die biologische Wirksamkeit der erfindungsgemäßen Verbindungen soll anhand der folgenden Beispiele erläutert werden.

Herstellungsbeispiele

Beispiel 1

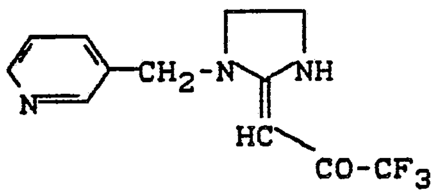

(Verfahren (a))

2,16 g (0,01 Mol) 1,1-Dimethylthio-4,4,4-trifluor-1-buten-3-on und 1,5 g (0,01 Mol) N-[Pyrid-3-yl)-methyl]-ethylendiamin werden in 20 ml Xylol 2 Stunden unter Rückfluß erhitzt. Die heiße Lösung wird filtriert und der beim Abkühlen auskristallierende Feststoff abgenutscht.

Man erhält so 1,7 g (63 % der Theorie) 1-(Pyrid-3-yl)methyl-2-(trifluormethylcarbonylmethylen)-imidazolidin als roter Feststoff vom Schmelzpunkt 157 °C.

Beispiel 2

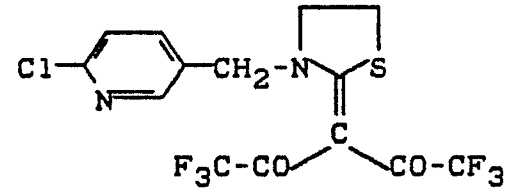

(Verfahren (b))

Zu einer Lösung von 4,8 g (0,015 Mol) 3-(6-Chlor-pyrid-3-yl)-methyl-2-(trifluormethylcarbonylmethylen)-1,3-thiazolidin und 2,4 g (0,03 Mol) Pyridin in 50 ml Dichlormethan werden bei 20 °C - 25 °C 6,3 g (0,03 Mol) Trifluoressigsäureanhydrid zugetropft und die Mischung 15 Stunden bei 20 °C gerührt. Anschließend wird das Lösungsmittel im Vakuum abdestilliert und der Rückstand unter Rühren mit Wasser versetzt. Das ausgefallene Produkt wird abgenutscht, mit Wasser gewaschen und getrocknet.

Man erhält so 5,1 g (80 % der Theorie) 2-[(Di-trifluormethylcarbonyl)-methylen]-3-(6-chlor-pyrid-3-yl)-methyl-1,3-thiazolidin als beiger Feststoff vom Zersetzungspunkt 180 °C.

Beispiel 3

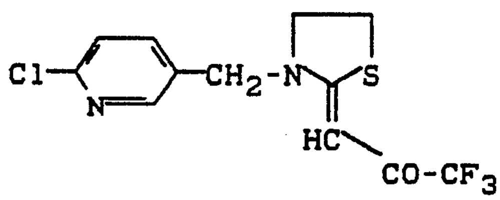

(Verfahren (c))

3,9 g (0,02 Mol) 2-Trifluormethylcarbonylmethylen-1,3-thiazolidin werden in 10 ml Tetrahydrofuran und 20 ml Dimethylformamid bei 25 °C portionsweise mit 0,72 g (0,024 Mol) Natriumhydrid (80 %-ig in Paraffin) versetzt und 15 Minuten auf 70 °C erhitzt. Nach dem Abkühlen werden bei 25 °C 3,2 g (0,02 Mol) 2-Chlor-5-chlormethyl pyridin in 20 ml Tetrahydrofuran zugegeben, 30 Stunden bei 60 °C und 15 Stunden unter Rückfluß erhitzt. Anschließend wird eingeengt und der Rückstand mit Wasser verrührt. Das ausgefallene Produkt wird abgesaugt und getrocknet.

Man erhält 5,5 g (85,2 % der Theorie) 3-(6-Chlor-pyrid-3-yl)-methyl-2-(trifluormethylcarbonylmethylen)-1,3-thiazolidin vom Schmelzpunkt 101 °C.

Beispiel 4

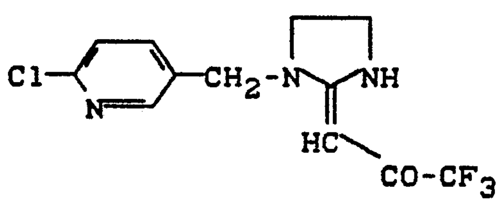

(Verfahren (d/α))

12,6 g (0,06 Mol) 1-(6-Chlor-pyrid-3-yl)-methyl-2-methyl-2-imidazolin und 9,5 g (0,12 Mol) Pyridin in 120 ml Dichlormethan werden bei 20 °C tropfenweise mit 25,2 g (0,12 Mol) Trifluoressigsäureanhydrid versetzt

und 4 Stunden bei 25 °C gerührt. Anschließend wird eingeengt, mit 300 ml Wasser und 15,5 g (0,18 Mol) Natriumhydrogencarbonat versetzt. Die Mischung wird nach beendeter Zugabe 3 Stunden nachgerührt und das ausgefallene Produkt abgesaugt.

Man erhält 16,4 g (89,5 % der Theorie) 1-(6-Chlor-pyrid-3-yl)-methyl-2-(trifluormethylcarbonylmethylen)-imidazolidin vom Schmelzpunkt 135 °C.

Beispiel 5

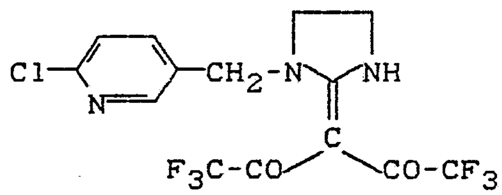

(Verfahren (d/β),

Zu einer Lösung von 2,1 g (0,01 Mol) 1-(6-Chlor-pyrid-3-yl)-methyl-2-methyl-imidazolin und 0,97 g (0,01 Mol) Pyridin in 30 ml Dichlormethan werden 25 °C 6,3 g (0,03 Mol) Trifluoressigsäureanhydrid zugetropft und 15 Stunden bei 25 °C gerührt. Das Reaktionsgemisch wird eingeengt, der Rückstand mit 100 ml Wasser und portionsweise mit 5,0 g (0,06 Mol) Natriumhydrogencarbonat versetzt. Das ausgefallene Produkt wird abgesaugt und getrocknet.

Man erhält 3,0 g (78 % der Theorie) 2-[(Di-trifluormethylcarbonyl)-methylen]-3-(6-chlor-pyrid-3-yl)-methylimidazolidin vom Schmelzpunkt 181 °C.

Beispiel 6

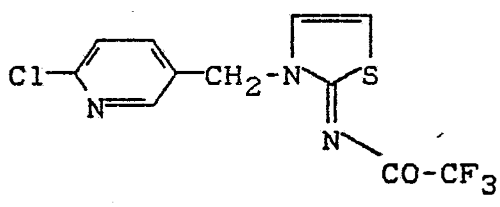

(Verfahren (e))

Zu einer Suspension von 2,6 g (0,01 Mol) 2-Amino-3-(6-chlor-pyrid-3-yl)-methyl-thiazoliumchlorid in 50 ml Pyridin werden bei 25 °C 2,1 g (0,01 Mol) Trifluoressigsäureanhydrid zugetropft. Das Gemisch wird 24 Stunden bei 20 °C gerührt und anschließend eingeengt. Der Rückstand wird mit 50 ml Wasser versetzt, zweimal mit Dichlormethan extrahiert, die vereinigten Extrakte über Natriumsulfat getrocknet und das Lösungsmittel im Vakuum verdampft. Der ölige Rückstand wird über eine Kieselgelsäule (Chloroform/Ethanol = 19 : 1) gereinigt.

Man erhält 0,63 g (19,5 % der Theorie) 3-(6-Chlor-pyrid-3-yl)-methyl-2-trifluormethylcarbonylimino-4-thiazolin vom Schmelzpunkt 114 - 117 °C.

Analog Beispiele 1 bis 6 bzw. Verfahren (a) bis (e) können die folgenden Verbindungen der Formel (I) herstellt werden:

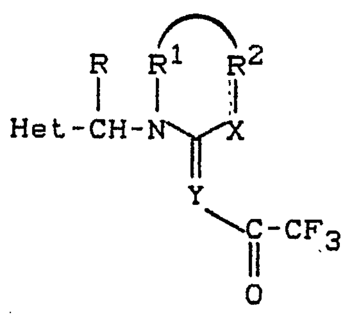

(I)

Tabelle 7

| Beisp.-Nr. | Het | R | 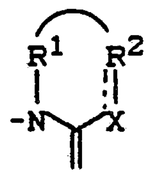 | Y | Physikal. Konstante |
|---|---|---|---|---|---|
| 7 | 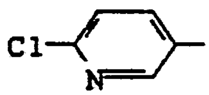 | H | 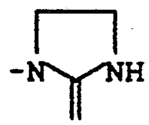 | N | Fp. 83° C |
| 8 | 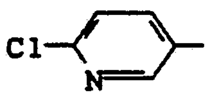 | H | 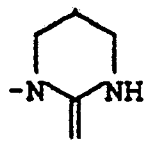 | CH | Fp. 154° C |
| 9 | 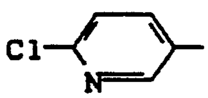 | H | 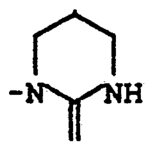 | $C-CO-CF_3$ | Fp. 204° C |
| 10 | 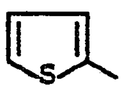 | H | 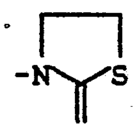 | CH | Fp. 87° C |
| 11 | 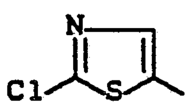 | H | 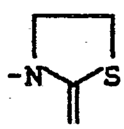 | CH | *δ=5,79(s) |
| 12 | 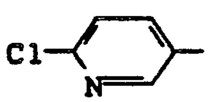 | H | 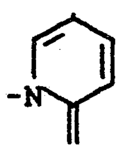 | N | Fp.60-62° C |

* Sind die δ-Werte der $^1$H-NMR-Spektren, die in $CDCl_3$ aufgenommen wurden. Angegeben sind die chemischen Verschiebungen für die Gruppierung 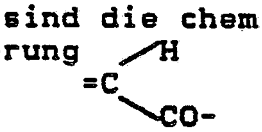

Ausgangsverbindung der Formel (III)

Beispiel (III-1)

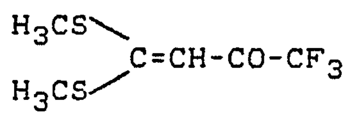

Zu einer Suspension von 2,4 g (0,1 Mol) Natriumhydrid (80 %-ig) in Paraffin in 100 ml wasserfreiem Dimethylformamid werden bei 5 °C - 10 °C 5,6 g (0,05 Mol) 1,1,1-Trifluoraceton getropft. Die Mischung wird 20 Minuten ohne Kühlung gerührt und dann mit 20 ml Schwefelkohlenstoff tropfenweise versetzt. Unter Gasentwicklung färbt sich der Reaktionsansatz dunkelrot, die Innentemperatur steigt auf 45 °C an. Es wird 2 Stunden bei 45 °C - 50 °C gerührt, anschließend auf 0 °C bis 5 °C gekühlt und bei maximal 5 °C mit 14,2 g (0,1 Mol) Iodmethan versetzt. Nach 16 stündigem Rühren bei 25 °C wird der Ansatz in 300 ml Eiswasser eingetragen und 2 mal mit je 100 ml Dichlormethan extrahiert. Die organische Phase wird über Natriumsulfat getrocknet, das Lösungsmittel im Vakuum verdampft und der verbleibende Rückstand mit Ether verrührt. Die Etherlösung wird zweimal mit Aktivkohle aufgekocht, filtriert, und auf ein Volumen von ca. 50 ml eingeengt. Das Produkt kristallisiert nach Zugabe von 100 ml Petrolether aus.

Man erhält 3,2 g (29,6 % der Theorie) 1,1-Bismethylthio-4,4,4-trifluor-1-buten-3-on als blaßblaue Kristalle vom Schmelzpunkt 96 °C.

Ausgangsverbindung der Formel (Va)

Beispiel (Va-1)

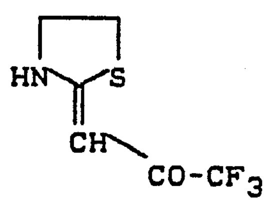

Zu einer Lösung von 6,1 g (0,06 Mol) 2-Methyl-2-thiazolin und 9,5 g (0,12 Mol) Pyridin in 60 ml Dichlormethan werden bei 20 °C 25,2 g (0,12 Mol) Trifluoressigsäureanhydrid tropfenweise gegeben. Die Mischung wird 4 Stunden bei 25 °C gerührt, dann das Lösungsmittel im Vakuum abdestilliert und zum Rückstand ca. 100 ml Wasser gegeben. Unter Rühren gibt man nach und nach 15,5 g (0,18 Mol) Natriumhydrogencarbonat zu und sammelt den ausgefallenen Feststoff auf einer Nutsche.

Man erhält 9,1 g (77 % der Theorie) 2-Trifluormethylcarbonylmethylen-thiazolidin als beigefarbener Feststoff vom Schmelzpunkt 127 °C.

Ausgangsverbindung der Formel (VII)

Beispiel (VII-1)

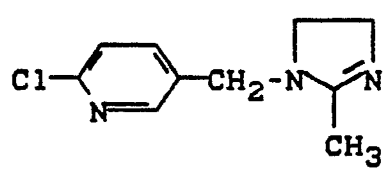

11,1 g (0,06 Mol) N-[(6-Chlor-pyrid-3-yl)-methyl]-ethylendiamin, 7,2 g (0,06 Mol) Orthoessigsäuretyrimethylester und 0,1 g p-Toluolsulfonsäure in 60 ml Xylol werden 8 Stunden unter Rückfluß erhitzt. Die Mischung wird filtriert, das Lösungsmittel im Vakuum abdestilliert und der Rückstand im

Vakuum destilliert.

Man erhält 11,0 g (88 % der Theorie) 1-(6-Chlor-pyrid-3-yl)-methyl-2-methyl-2-imidazolin vom Siedepunkt Kp.: 110 °C - 115 °C/202 Hektopascal.

Ausgangsverbindungen der Formel (VIII)

Beispiel (VIII-1)

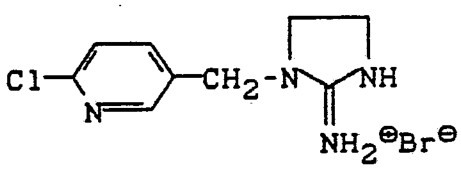

Zu einer Lösung von 5,55 g (0,03 Mol) N-[(6-Chlor-pyrid-3-yl)-methyl]-ethylendiamin in 50 ml Methanol werden bei 20 °C - 25 °C 3,2 g Bromcyan in 10 ml Methanol zugetropft. Nach beendeter Zugabe wird 2 Stunden bei 50 °C gerührt dann auf 20 °C abgekühlt und das ausgefallene Produkt auf einer Nutsche gesammelt.

Man erhält 7,2 g (83 % der Theorie) 1-(6-Chlor-pyrid-3-yl)-methyl-2-imino-imidazolin-Hydrobromid vom Zersetzungspunkt 225 °C.

Beispiel (VIII-2)

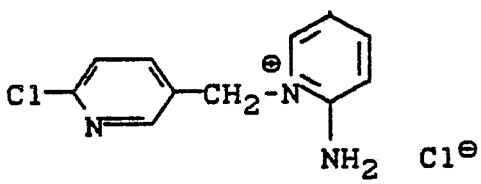

9,6 g 2-Aminopyridin und 16,2 g 2-Chlor-5-chlormethyl-pyridin in 100 ml Ethanol 8 Stunden unter Rückfluß erhitzt. Nach dem Abkühlen, wird abgesaugt, mit Ethanol gewaschen und getrocknet.

Man erhält 20 g 2-Amino-1-(6-Chlor-pyrid-3-yl)-methyl-pyridiniumchlorid vom Schmelzpunkt 220 °C - 225 °C.

Beispiel (VIII-3)

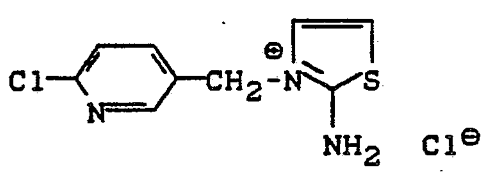

10 g 2-Aminothiazol und 16,2 g 2-Chlor-5-chlormethyl-pyridin in 100 ml Ethanol werden 8 Stunden unter Rückfluß erhitzt. Nach dem Abkühlen, wird abgesaugt, mit Ethanol gewaschen und getrocknet.

Man erhält 23 g 2-Amino-3-(6-chlor-pyrid-3-yl)-methyl-thiazoliumchlorid vom Schmelzpunkt 210 °C - 215 °C.

Verwendungsbeispiele

In den folgenden Verwendungsbeispielen wurde die folgende Verbindung als Vergleichssubstanz eingesetzt:

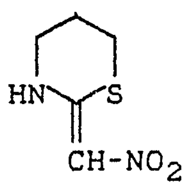

Tetrahydro-2-nitromethylen-2H-1,3-thiazin aus US-PS 3 993 648.

Beispiel A

Nephotettix-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test zeigen z. B. die folgenden Verbindungen der Herstellungsbeispiele (3) und (5) bei einer Wirkstoffkonzentration von 0,0001 % nach 6 Tagen eine Wirkung von 90 % und 100 %, während die Vergleichssubstanz (A) eine Wirkung von 20 % zeigt.

Beispiel B

Aphis-Test (systemische Wirkung)

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Aphis fabae) befallen sind, angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z. B. die folgenden Verbindungen der Herstellungsbeispiele (4), (5) und (8) bei einer Wirkstoffkonzentration von 0,01 % nach 4 Tagen eine Wirkung von 100 %, während die Vergleichssubstanz (A) eine Wirkung von 50 % zeigt.

Beispiel C

Grenzkonzentrations-Test / Wurzelsystemische Wirkung Testinsekt: Phaedon cochleariae-Larven
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/1) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z. B. die folgenden Verbindungen der Herstellungsbeispiele (3) und (8) bei einer Wirkstoffkonzentration von 20 ppm eine Wirkung von 100 %, während die Vergleichssubstanz (A) keine Wirkung zeigt.

Beispiel D

Grenzkonzentrations-Test / Wurzelsystemische Wirkung Testinsekt: Myzus persicae
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/1) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z. B. die folgenden Verbindungen der Herstellungsbeispiele (3), (4), (5) und (8) bei einer Wirkstoffkonzentration vom 20 ppm eine Wirkung von 100 %, während die Vergleichssubstanz (A) keine Wirkung zeigt.

Beispiel E

Test mit Lucilia cuprina resistent-Larven

Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird der Abtötungsgrad bestimmt.

Bei diesem Test zeigen z.B. die folgenden Verbindungen der Herstellungsbeispiele (4), (5), (8) und (9) bei einer Wirkstoffkonzentration von 100 ppm eine Abtötung von 100 %.

## Ansprüche

1. Trifluormethylcarbonyl-Derivate der Formel I

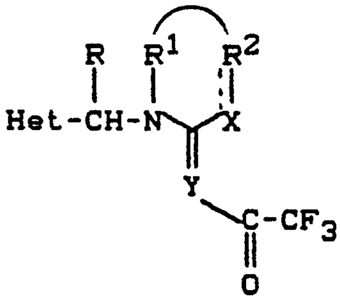

(I)

in welcher
Het für gegebenenfalls einfach oder mehrfach substituiertes Hetaryl steht,
R für Wasserstoff oder $C_1$-4-Alkyl steht,
X für Sauerstoff, Schwefel, Stickstoff oder für die
Gruppierungen >NH oder $=CR^4$ steht,
worin
$R^4$ für Wasserstoff oder Alkyl steht,
Y für Stickstoff oder den Rest $=CR^3$ steht,
worin
$R^3$ für Wasserstoff oder den Rest $-CO-CF_3$ steht und
$R^1$ und $R^2$ gemeinsam mit dem angrenzenden Stickstoffatom und dem Rest X für einen heterocyclischen Rest der Formel

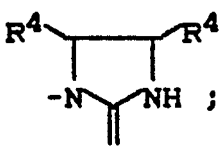 ; 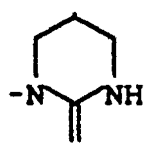 ; 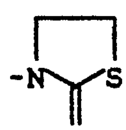 ; 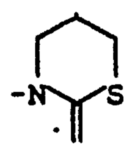 ;

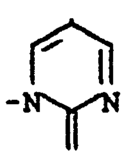 ; 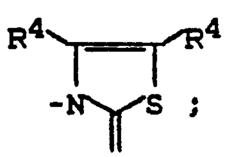 ; 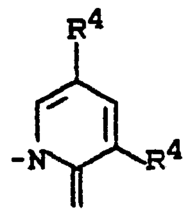 ; 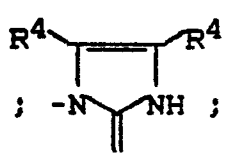 ;

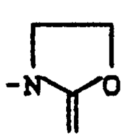 oder 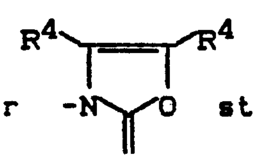 stehen, worin

$R^4$ die oben angegebene Bedeutung besitzt.

2. Trifluormethylcarbonyl-Derivate gemäß Formel (I) in Anspruch 1, in welcher
Het für gegebenenfalls durch Fluor, Chlor, Brom, Cyano, Nitro, $C_1$-$C_4$-Alkyl, $C_1$-$C_4$-Alkoxy, $C_1$-$C_4$-Alkylthio, Halogen-$C_1$-$C_4$-alkyl, Halogen-$C_1$-$C_4$-alkoxy, Halogen-$C_1$-$C_4$-alkylthio, Amino, $C_1$-$C_4$-Alkylamino oder Di-$C_1$-$C_4$-

alkylamino substituiertes Furyl, Thiophenyl, Pyrazolyl, Imidazolyl, Pyrrolyl, 1,2,4-Triazolyl, 1,2,3-Triazolyl, Pyrimidinyl, Pyrazinyl, Pyridyl, Oxazolyl, Isoxazolyl, 1,2,4-Oxadiazolyl, 1,3,4-Oxadiazolyl, Thiazolyl, Isothiazolyl, 1,2,5-Thiadiazolyl und 1,3,4-Thiadiazolyl steht,

R für Wasserstoff oder Methyl steht,

X für Sauerstoff, Schwefel, Stickstoff oder für die Gruppierungen >NH oder >=C$R^4$ steht,

Y für Stickstoff oder den Rest >=C$R^3$ steht,

worin

$R^3$ für Wasserstoff oder den Rest $-CO-CF_3$ steht und

$R^1$ und $R^2$ gemeinsam mit dem angrenzenden Stickstoffatom und dem Rest X für einen heterocyclischen Rest der Formel

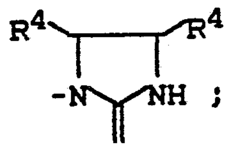 ; 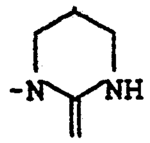 ; 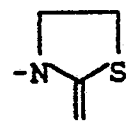 ; 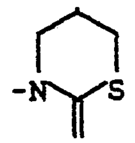 ;

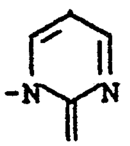 ; 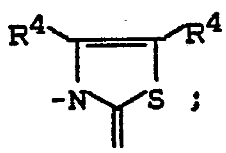 ; 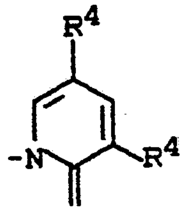 ; 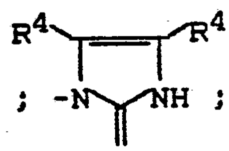 ;

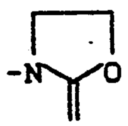 oder 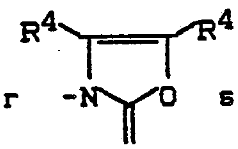 stehen, worin

$R^4$ für Wasserstoff oder $C_1$-$C_4$-Alkyl steht.

3. Trifluormethylcarbonyl-Derivate der Formel I gemäß Anspruch 1, in welcher

Het für gegebenenfalls durch Fluor, Chlor, Brom, Cyano, Nitro, Methyl, Ethyl, Methoxy, Methylthio, Trifluormethyl, Trifluormethoxy, Trifluormethylthio, Dimethylamino oder Diethylamino substituiertes Thiophenyl, Pyrazolyl, 1,2,4-Triazolyl, Pyrazinyl, Pyrimidinyl, Pyridyl, Isoxazolyl, Oxazolyl, Thiazolyl, 1,2,5-Thiadiazolyl oder 1,3,4-Thiadiazolyl steht,

R für Wasserstoff steht,

X für Sauerstoff, Schwefel, Stickstoff oder für die Gruppierungen

>NH oder >=C$R^4$ steht,

worin

$R^4$ für Wasserstoff steht,

Y für Stickstoff oder den Rest >=C$R^3$ steht,

worin

$R^3$ für Wasserstoff oder den Rest $-CO-CF_3$ steht und

$R^1$ und $R^2$ gemeinsam mit dem angrenzenden Stickstoffatom und dem Rest X für einen heterocyclischen Rest der Formel

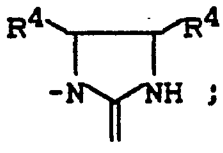 ; 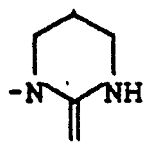 ; 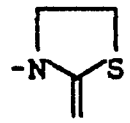 ; 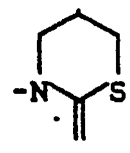 ;

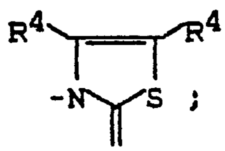 ; 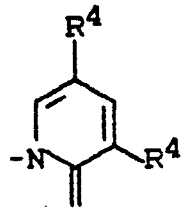 ; 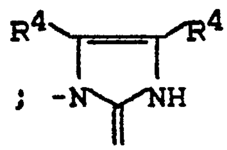

oder 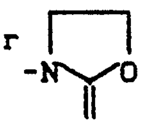

stehen, worin $R^4$ die oben angegebene Bedeutung hat.

4. Verfahren zur Herstellung von Trifluormethylcarbonyl-Derivaten der Formel I

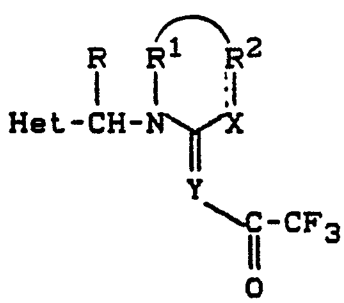

(I)

in welcher
Het für gegebenenfalls einfach oder mehrfach substituiertes Hetaryl steht,
R für Wasserstoff oder $C_1$-$C_4$-Alkyl steht,
X für Sauerstoff, Schwefel, Stickstoff oder für die Gruppierungen $>NH$ oder $=CR^4$ steht,
Y für Stickstoff oder den Rest $=CR^3$ steht,
worin
$R^3$ für Wasserstoff oder den Rest -CO-$CF_3$ steht und
$R^1$ und $R^2$ gemeinsam mit dem angrenzenden Stickstoffatom und dem Rest X für einen heterocyclischen Rest der Formel

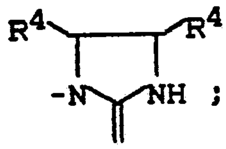 ; 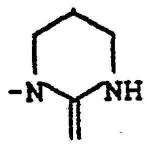 ; 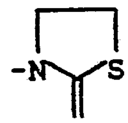 ; 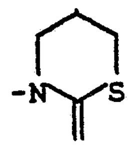 ;

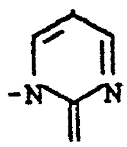 ; 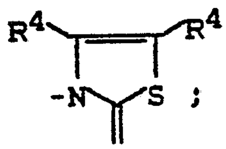 ; 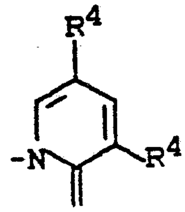 ; 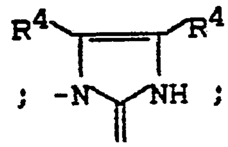 ;

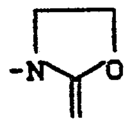 oder 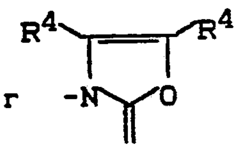

stehen, worin
$R^4$ für Wasserstoff oder Alkyl steht,
dadurch gekennzeichnet,
(a) daß man zum Erhalt von Verbindungen der Formel (Ia)

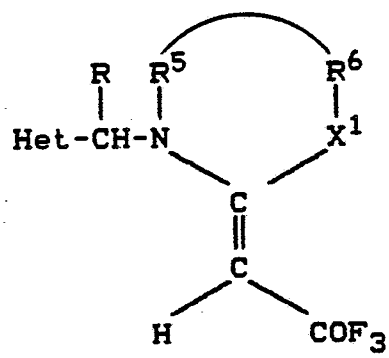

(Ia)

in welcher
Het und R die oben angegebene Bedeutung besitzen,
$R^5$ und $R^6$ gemeinsam für einen gegebenenfalls durch $C_1$-$C_4$-Alkyl substituierten $C_2$-$C_3$-Alkylen-rest stehen und
$X^1$ für Sauerstoff, Schwefel oder die Gruppierung >NH steht,
Hetarylmethylamine der Formel (II)

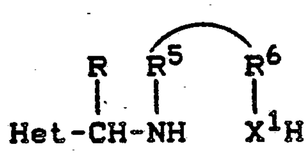

(II)

in welcher
Het, R, $R^5$, $R^6$ und $X^1$ die oben angegebenen Bedeutungen haben,
mit 1,1-Bismethylthio-4,4,4-trifluor-1-buten-3-on der Formel (III)

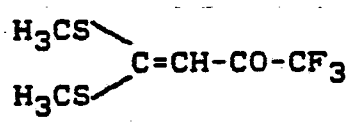

(III)

gegebenenfalls in Gegenwart von Verdünnungsmitteln umsetzt, oder
(b) daß man zum Erhalt von Verbindungen der Formel (Ib)

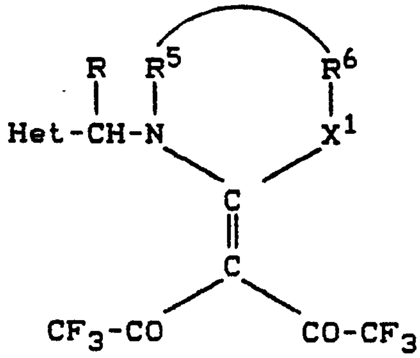

(Ib)

in welcher
Het, R, $R^5$, $R^6$ und $X^1$ die oben angegebene Bedeutung haben,
Trifluormethylcarbonyl-Derivate der Formel (Ia)

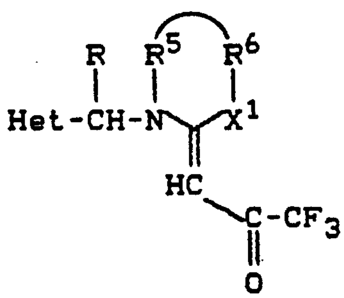

(Ia)

in welcher
Het, R, $R^5$, $R^6$ und $X^1$ die oben angegebenen Bedeutungen haben,
mit einem Trifluormethylcarbonsäure-Derivat der Formel (IV)

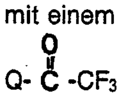 (IV)

in welcher
Q für Fluor, Chlor oder den Rest -O-CO-$CF_3$ steht,
gegebenenfalls in Gegenwart eines Säureakzeptors und gegebenenfalls in Gegenwart eines Verdünnungsmittels umsetzt,
oder
(c) daß man zum Erhalt von Verbindungen der Formel (Ic)

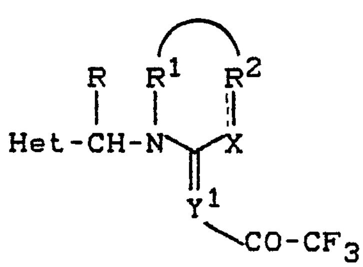

(Ic)

in welcher
Het, R, $R^1$, $R^2$, X die oben angegebene Bedeutung besitzen und
$Y^1$ für die Gruppierungen =CH oder -C-CO-$CF_3$ steht,
Verbindungen der Formel (V)

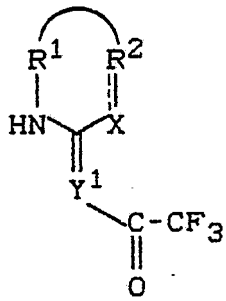

(V)

in welcher
$R^1$, $R^2$ und X die oben angegebenen Bedeutungen haben und
$Y^1$ für die Gruppierung $\geqq$CH oder $\geqq$C-CO-$CF_3$ steht, mit Halogeniden der Formel (VI)

Het-$\overset{R}{C}$H-Hal (VI)

in welcher
Het und R die oben angegebenen Bedeutungen haben und
Hal für Halogen steht,
gegebenenfalls in Gegenwart einer starken Base und gegebenenfalls in Gegenwart eines Verdünnungsmittels umsetzt,
oder
(d) daß man zum Erhalt von Verbindungen der Formel (Id)

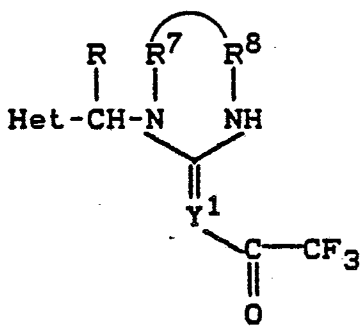

(Id)

in welcher
Het, R und $Y^1$ die oben angegebene Bedeutung haben und
$R^7$ und $R^8$ für einen gegebenenfalls durch $C_1$-$C_4$-Alkyl substituierten $C_2$-$C_3$-Alkylen-oder Alkenylen-Rest stehen,
Methyl-Derivate der Formel (VII)

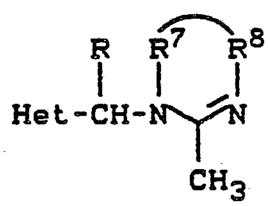

(VII)

in welcher
Het, R, $R^7$ und $R^8$ die oben angegebenen Bedeutungen haben
(α) mit der zweifachen molaren Menge Trifluormethylcarbonsäure-Derivat der Formel (IV)

Q-$\overset{O}{\overset{\|}{C}}$-$CF_3$ (IV)

in welcher
Q die oben angegebenen Bedeutungen hat,
gegebenenfalls in Gegenwart eines Säureakzeptors und gegebenenfalls in Gegenwart eines Verdünnungsmittels zu den Trifluormethylcarbonyl-Derivaten der Formel (Id-α)

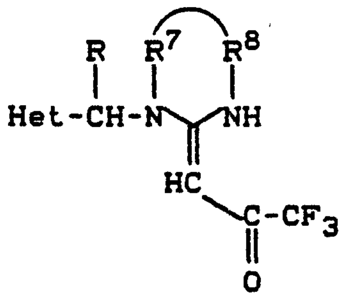

(Id-α)

in welcher
Het, R, $R^7$ und $R^8$ die oben angegebenen Bedeutungen haben,
umsetzt, oder
(β) mit der dreifachen molaren Menge Trifluormethylcarbonsäure-Derivat der Formel (IV)

$Q-\overset{O}{\overset{\|}{C}}-CF_3$ (IV)

in welcher
Q die oben angegebenen Bedeutungen hat,
gegebenenfalls in Gegenwart eines Säureakzeptors und gegebenenfalls in Gegenwart eines Verdünnungsmittels zu den Trifluormethylcarbonyl-Derivaten der Formel (Id-β)

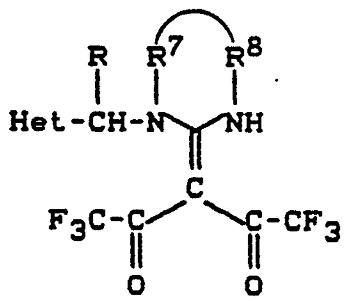

(Id-β)

in welcher
Het, R, $R^7$ und $R^8$ die oben angegebenen Bedeutungen haben,
umsetzt, oder
(e) zum Erhalt von Verbindungen der Formel (Ie)

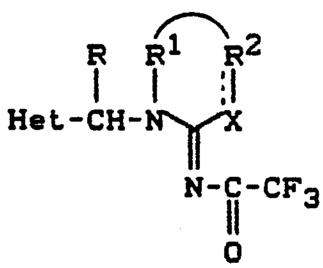

(Ie)

in welcher
Het, R, $R^1$, $R^2$, X die oben angegebene Bedeutung haben,
Salze der Formel (VIII)

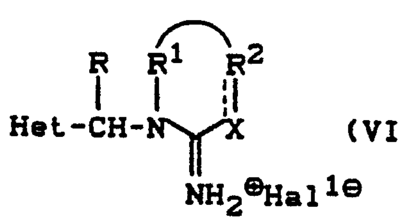

(VIII)

in welcher
Het, R, $R^1$, $R^2$ und X die oben angegebenen Bedeutungen haben und
$Hal^1$ für Halogen steht,
mit einem Trifluormethylcarbonsäure-Derivat der Formel (IV)

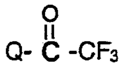 (IV)

in welcher
Q die oben angegebene Bedeutung hat,
gegebenenfalls in Gegenwart eines Säureakzeptors und gegebenenfalls in Gegenwart eines Verdünnungsmittels umsetzt.

5. Schädlingsbekämpfungsmittel, gekennzeichnet durch einen Gehalt an mindestens einem Trifluormethylcarbonyl-Derivat der Formel (I).

6. Insektizide und ektoparasitizide Mittel, gekennzeichnet durch einen Gehalt an mindestens einem Trifluormethylcarbonyl-Derivat der Formel (I).

7. Verfahren zur Bekämpfung von Insekten und/oder Ektoparasiten, dadurch gekennzeichnet, daß man Tri fluormethylcarbonyl-Derivate der Formel (I) auf Insekten und/oder Ektoparasiten und/oder deren Lebensraum einwirken läßt.

8. Verwendung von Trifluormethylcarbonyl-Derivaten der Formel (I) zur bekämpfung von Insekten und/oder Ektoparasiten.

9. Verfahren zur Herstellung von Schädlingsbekämpfungsmitteln, dadurch gekennzeichnet, daß man Trifluormethylcarbonyl-Derivate der Formel (I) mit Streckmitteln und/oder oberflächenaktiven Mitteln vermischt.

10. 1,1-Bismethylthio-4,4,4-trifluor-1-buten-3-on der Formel

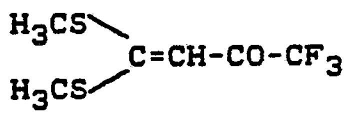 (III)

11. Trifluormethylcarbonyl-Derivate der Formel (Va)

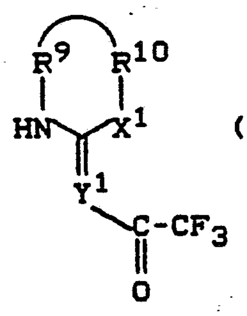 (Va)

in welcher
$Y^1$ für die Gruppierungen $\geqq$CH oder $\geqq$C-CO-$CF_3$ steht
$X^1$ für Sauerstoff, Schwefel oder für die Gruppierung $>$NH steht und
$R^9$ und $R^{10}$ gemeinsam mit dem angrenzenden Stickstoffatom und den Rest $X^1$ für einen Heterocyclus der Formel

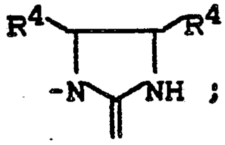 ; 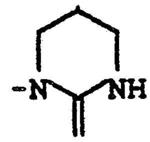 ; 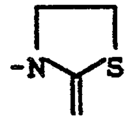 ; 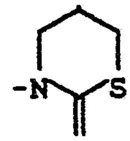 ;

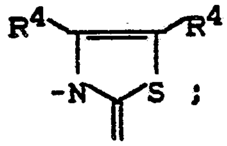 ; 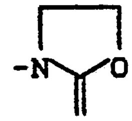 oder 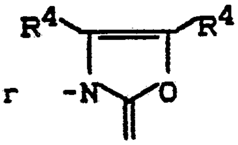

stehen,
worin
$R^4$ für Wasserstoff oder Alkyl steht.

12. Verbindungen der Formel (VIII)

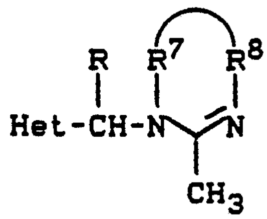

(VII)

in welcher
Het für gegebenenfalls einfach oder mehrfach substituiertes Hetaryl steht,
R für Wasserstoff oder $C_1$-$C_4$-Alkyl steht,
$R^7$ und $R^8$ für einen gegebenenfalls durch $C_1$-$C_4$-Alkyl substituierten $C_2$-$C_3$-Alkylen-oder Alkenylen-Rest stehen.